# EUROPEAN PATENT SPECIFICATION

(11) **EP 2 718 436 B1**
(45) Date of publication and mention of the grant of the patent: **28.03.2018**
(21) Application number: 12728113.7
(22) Date of filing: 08.06.2012
(51) Int. Cl.: C12N 15/113, A61K 31/713, A61P 35/00

(54) **MATERIALS AND METHODS FOR TREATING PTEN MUTATED OR DEFICIENT CANCER**
MATERIALIEN UND VERFAHREN ZUR BEHANDLUNG VON PTEN-MUTIERTEM ODER -DEFIZIENTEM KREBS
SUBSTANCES ET PROCÉDÉS POUR TRAITER UN CANCER À DÉFICIENCE OU À MUTATION DU GÈNE PTEN

(30) Priority: 10.06.2011 GB 201109966; 10.06.2011 US 201161495602 P
(43) Date of publication of application: 16.04.2014
(73) Proprietor: The Institute of Cancer Research: Royal Cancer Hospital, London Greater London SW7 3RP (GB)
(72) Inventor: ASHWORTH, Alan, London Greater London SW7 3RP (GB); LORD, Christopher James, London Greater London SW7 3RP (GB); BROUGH, Rachel, London Greater London SW7 3RP (GB); FRANKUM, Jessica, London Greater London SW7 3RP (GB)
(74) Representative: Kiddle, Simon John
(86) International application number: PCT/GB2012/051290
(87) International publication number: WO 2012/168721

(56) References cited:
- WO-A1-2012/000103
- MIN SUP SONG ET AL.: "Nuclear PTEN regulates the APC-CDH1 tumor-suppressive complex in a phosphatase-independent manner", CELL, CELL PRESS, US, vol. 144, no. 2, 15 December 2010 (2010-12-15), pages 187-199, XP028152918, ISSN: 0092-8674, DOI: 10.1016/J.CELL.2010.12.020 [retrieved on 2010-12-17] -& Min Sup Song et al.: "Supplemental Information", , 21 January 2011 (2011-01-21), XP002682282, Retrieved from the Internet: URL:http://www.sciencedirect.com/science/a rticle/pii/S009286741001473X [retrieved on 2012-08-23]
- HARRINGTON E A ET AL: "VX-680, a potent and selective small molecule inhibitor of the Aurora kinases, suppresses tumor growth in vivo", NATURE MEDICINE, NATURE PUBLISHING GROUP, NEW YORK, NY, US, vol. 10, no. 3, 1 March 2004 (2004-03-01), pages 262-267, XP002490468, ISSN: 1078-8956, DOI: 10.1038/NM1003
- PARK ET AL: "Combination of PTEN and gamma-Ionizing Radiation Enhances Cell Death and G2/M Arrest Through Regulation of AKT Activity and p21 Induction in Non-Small-Cell Lung Cancer Cells", INTERNATIONAL JOURNAL OF RADIATION: ONCOLOGY BIOLOGY PHYSICS, PERGAMON PRESS, USA, vol. 70, no. 5, 25 March 2008 (2008-03-25) , pages 1552-1560, XP022553483, ISSN: 0360-3016, DOI: 10.1016/J.IJROBP.2007.11.069

## Description

### Field of the Invention

The present invention relates to materials and methods for treating PTEN mutated or deficient cancer using mitotic kinase inhibitors, and to methods of screening for agents for treating PTEN mutated or deficient cancer.

### Background of the Invention

Central to the design of novel therapeutic strategies for cancer is the identification of genes that are critical to the survival of tumor cells but which are largely redundant in normal cells. Correlating molecular changes with tumorigenesis has provided one route to the identification of potential drug targets and provides the rationale behind efforts to characterise genetic variation and gene expression in tumors. However, the correlative nature of these data means that it is frequently not possible to determine whether the observations are causative or merely an effect of the disease state. For example, tumor cells generally exhibit anywhere between 10⁴ and 10⁵ genetic changes compared to germline DNA but theoretical estimates suggest that only a few of these mutations (probably less than 10) are critical drivers of tumor formation and survival (reviewed in (1)).

Brough et al .(Current Opinion in Genetics & Development 2011, 21:34-41) describe how the biological concept of synthetic sickness/lethality (SSL) may be used to identify new therapeutic approaches and targets in models from yeast through to human cells. It is known to target genes that are overexpressed in particular forms of cancer. However, despite these successes, developing drugs that selectively kill cancer cells without harming normal cells remains a considerable challenge. This process is particularly problematic when considering how to target a protein such as a dysfunctional tumour suppressor that is either largely inactive or even completely absent. If one gene in an SSL relationship is a tumour suppressor gene, then its synthetic lethal partner becomes a candidate therapeutic target that could be used in tumour cells with a defined tumour suppressor gene dysfunction. SSL can occur between genes acting in the same biochemical pathway or in distinct but compensatory pathways.

WO 2012/000103 relates to targeting cancers with PTEN mutations with PLK4 antagonists. Harrington et al. (Nature Medicine, 10(3): 262-267, 2004) describes VX-680, a small molecule inhibitor of Aurora kinases. Song et al. (Cell, 144: 187-199, 2011) relates to PLK1 and Aurora kinases as targets for inhibition in the treatment of PTEN mutated cancer. WO2010/007756 describes a series of compounds for inhibiting TTK
for the treatment of cancer. US2011/0002923 describes the use of a TTK antagonists for treating HER-2 positive breast cancer specifically. US2003/0045491 describes a method for reducing growth of a cancerous cell by contacting it with an agent to reduce TTK activity. There is no mention of the use of TTK inhibitors in the treatment of PTEN mutated or deficient cancers in these documents.

In breast cancer, defining the critical genes involved in tumor cell survival can in some cases lead to the development of novel therapeutic approaches to the disease, the most notable recent example being the development of agents such as trastuzumab and lapatinib, that target the reliance of some breast tumors upon the oncogene ERBB2 (2). However, despite the wealth of molecular, genetic and histological characterization of tumors and cell line models, our understanding of the genetic dependencies in cancers such as breast cancer remains relatively poor. Accordingly, there is a need in the art for new therapeutic strategies, in particular those which target genetic dependencies of different forms of cancer by finding which of the large number of mutated genes in different forms of cancer are critical to tumor formation and survival.

### Summary of the Invention

Broadly, the present invention is based on work which attempts to comprehensively define the genetic dependencies for a set of potentially "druggable" genes in a wide range of tumor cell line models. In doing so, we not only reaffirm the impact of PI3-kinase and ERBB2 signalling in the breast cancer but importantly identify novel genetic dependencies for genetically defined subsets of cancer. In particular, the present invention is based on the experiments described herein in which inhibition of a mitotic kinase, such as TTK protein kinase, is effective for the treatment of PTEN mutated or deficient cancer.

Phosphatase and tensin homolog ("PTEN") is a gene that has been identified as a tumor suppressor through the action of its phosphatase product and is mutated in a large number of cancers at high frequency. The protein encoded this gene is a phosphatidylinositol-3,4,5-trisphosphate 3-phosphatase. It contains a tensin like domain as well as a catalytic domain similar to that of the dual specificity protein tyrosine phosphatases. Unlike most of the protein tyrosine phosphatases, this protein preferentially dephosphorylates phosphoinositide substrates. It negatively regulates intracellular levels of phosphatidylinositol-3,4,5-trisphosphate in cells and functions as a tumor suppressor by negatively regulating AKT/PKB and mTOR signalling pathways. The HUGO Gene Symbol report for PTEN can be found at
http://www.genenames.org/data/hgnc data.php?hgnc id=9588, which provides links to the PTEN nucleic acid and amino acid sequences, as well as reference to the homologous murine and rat proteins. Mitotic kinases are an established group of proteins involved in the regulation of cell checkpoints and division, see Nigg (Nature Reviews, 2: 21-31, 2001). The mitotic kinase that may be targeted in all aspects of the present is TTK.
set out in the following table:

| | **Query** | **Gene ID** | **Symbol** | **Name** | **UniProt ID** |
|---|---|---|---|---|---|
| 1 | AURKA | **ENSG00000087586** | AURKA | Serine/threonine-protein kinase 6 (EC 2.7.11.1)(Aurora kinase A) (Aurora-A) (Serine/threonine kinase 15)(Aurora/IPL1-related kinase 1)(Aurora-related kinase 1) (hARK1) (Breast tumor-amplified kinase) | **A3KFJ2 HUMAN** |
| 2 | TTK | **ENSG00000112742** | TTK | Dual specificity protein kinase TTK (EC 2.7.12.1) (Phosphotyrosine picked threonine-protein kinase) (PYT) | **A8K8U5_HUMAN** |
| 3 | CDK4 | **ENSG00000135446** | CDK4 | Cell division protein kinase 4 (EC 2.7.11.22) (Cyclin-dependent kinase 4)(PSK-J3) | **Q6LC83_HUMAN** |
| 4 | PLK4 | **ENSG00000142731** | PLK4 | Serine/threonine-protein kinase PLK4 (EC 2.7.11.21) (Polo-like kinase 4)(PLK-4) (Serine/threonine-protein kinase Sak) (Serine/threonine-protein kinase 18) | **Q15455 HUMAN** |
| 5 | BUB1B | **ENSG00000156970** | BUB1B | Mitotic checkpoint serine/threonine-protein kinase BUB1 beta (EC 2.7.11.1) (MAD3/BUB1-related protein kinase)(hBUBR1)(Mitotic checkpoint kinase MAD3L)(Protein SSK1) | **C9JLW5 HUMAN** |
| 6 | PLK1 | **ENSG00000166851** | PLK1 | Serine/threonine-protein kinase PLK1 (EC 2.7.11.21) (Polo-like kinase 1)(PLK-1) (Serine/threonine-protein kinase 13) (STPK13) | **PLK1 HUMAN** |
| 7 | CDC2 | **ENSG00000170312** | CDC2 | Cell division control protein 2 homolog (EC 2.7.11.22) (EC 2.7.11.23) (p34 protein. kinase) (Cyclin-dependent kinase 1) (CDK1) | **C9J497 HUMAN** |
| 8 | PLK3 | **ENSG00000173846** | PLK3 | Serine/threonine-protein kinase PLK3 (EC 2.7.11.21) (Polo-like kinase 3)(PLK-3) (Cytokine-inducible serine/threonine-protein kinase) (FGF-inducible kinase) (Proliferation-related kinase) | **PLK3_HUMAN** |
| 9 | AURKB | **ENSG00000178999** | AURKB | Serine/threonine-protein kinase 12 (EC 2.7.11.1) (Aurora-B)(Aurora-and Ipl1-like midbody-associated protein 1) (AIM-1) (Aurora/IPL1-related kinase 2)(Aurora-related kinase 2)(STK-1) | **B2RC06_HUMAN** |

A preferred example of a mitotic kinase, inhibitors of which can be used in accordance with the present invention for the treatment of PTEN mutated or deficient cancer, is TTK protein kinase. This kinase is a dual specificity protein kinase with the ability to phosphorylate tyrosine, serine and threonine. TTK is associated with cell proliferation and is essential for chromosome alignment at the centromere during mitosis and is required for centrosome duplication. It has been found to be a critical mitotic checkpoint protein for accurate segregation of chromosomes during mitosis. The HUGO Gene Symbol report for TTK can be found at
http://www.genenames.org/data/hgnc_data.php?hgnc_id=12401, which provides links to the TTK nucleic acid and amino acid sequences, as well as reference to the homologous murine and rat proteins.

According, in a first aspect the present invention provides an inhibitor of TTK protein kinase for use in a method of treating an individual having cancer by inhibiting said TTK protein kinase, wherein the cancer is a Phosphatase and Tensin Homolog (PTEN) mutated or deficient cancer.

In a further aspect, the present invention provides a method of screening for agents useful in the treatment of a PTEN mutated or deficient cancer, the method employing first and second cell lines, wherein the first cell line is PTEN and the second cell line is PTEN proficient, the method comprising:
(a) contacting the first and second mammalian cell lines with at least one candidate agent;
(b) determining the amount of cell death in the first and second cell lines;
(c) selecting a candidate agent which is synthetically lethal in the first cell line; and
(d) determining whether the candidate agent selected in step (c) is an inhibitor of TTK protein kinase.

In a further aspect, the present invention provides a method of screening for agents useful in the treatment of PTEN mutated or deficient cancer, the method comprising:
(a) contacting TTK protein kinase with at least one candidate agent;
(b) determining an effect of the at least one candidate agent on an activity of the TTK protein kinase; and
(c) selecting a candidate agent that inhibits the activity of the mitotic kinase as being useful for the treatment of the PTEN mutated or deficient cancer.

In some aspects, the present invention provides a method which comprises having identified a candidate agent useful for the treatment of a PTEN mutated or deficient cancer according to a method as described herein, the further step of manufacturing the compound in bulk and/or formulating the agent in a pharmaceutical composition.

The present invention includes the combination of the aspects and preferred features described except where such a combination is clearly impermissible or is stated to be expressly avoided. Embodiments of the present invention will now be described by way of example and not limitation with reference to the accompanying figures.

### Brief Description of the Figures

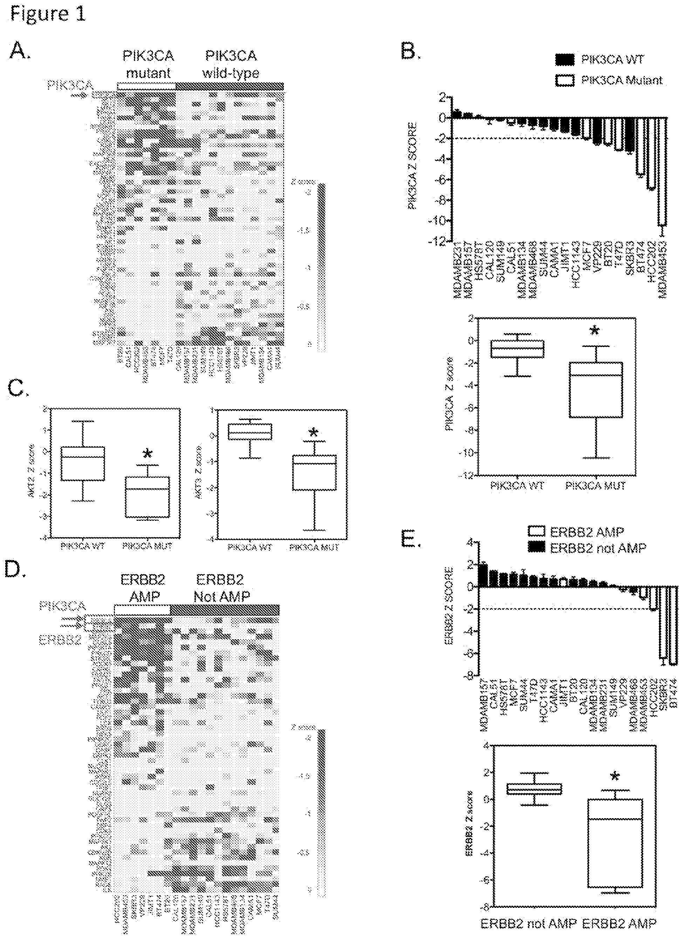

**Figure 1****. *PIK3CA* and *ERBB2* oncogene addiction effects in breast cancer confirmed by functional viability profiling. a.** Heat map showing the results of a supervised clustering of siRNA Z scores. Breast tumour cell lines were clustered according to *PIK3CA* gene mutation status and differential effects between *PIK3CA* mutant and wild type groups identified using the median permutation test. Statistically significant effects (p<0.05) are shown. For each siRNA, p values and the p<0.05 threshold are shown to the right of the heat map. siRNA targeting *PIK3CA* is marked by the arrow. **b.** Waterfall and box/whiskers plots of *PIK3CA* siRNA Z scores across the breast tumour cell line panel. In the box/whiskers plot, **p* = 0.014 between *PIK3CA* mutant and wild type groups using the median permutation test. **c.** Box/whiskers plots of *AKT2* and *AKT3* siRNA Z scores in *PIK3CA* mutant and wild type models (**p*= 0.047 and p 0.003 respectively using the median permutation test). **d.** Heat map showing the results of a supervised clustering of siRNA Z scores according to *ERBB2* amplification status in the breast tumour cell line panel. Statistically significant effects (*p*<0.05, median permutation test) are shown. *ERBB2* and *PIK3CA* siRNA are marked by arrows, **e.** Waterfall and box/whiskers plots of *ERBB2* siRNA Z scores across the breast tumour cell line panel. In the box/whiskers plot, **p* = 0.003 between *ERBB2* amplified and non-amplified groups using the median permutation test.

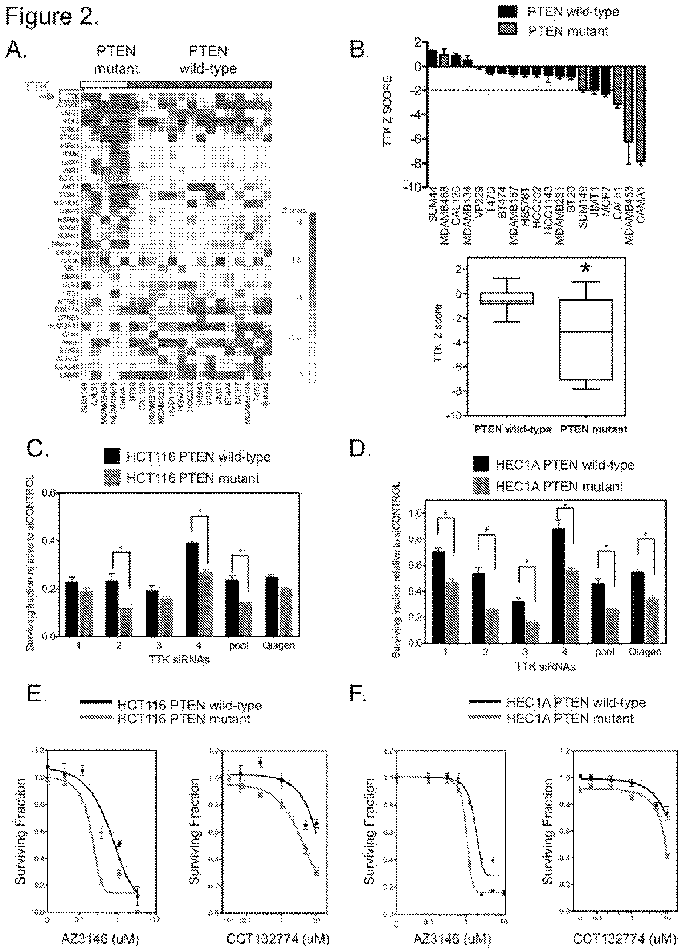

**Figure 2****. Inhibition of TTK is PTEN selective. a.** Heat map showing the results of a supervised clustering of siRNA Z scores. Breast tumour cell lines were clustered according to *PTEN* gene mutation status and differential effects between *PTEN* mutant and wild type groups identified using the median permutation test. Statistically significant effects (p<0.05) are shown. For each siRNA, p values and the p<0.05 threshold are shown to the right of the heat map. siRNA targeting *TTK* is marked by the arrow. **b.** Waterfall and box/whiskers plots of *TTK* siRNA Z scores across the breast tumour cell line panel. In the box/whiskers plot, **p* = 0.0012 between *PTEN* mutant and wild type groups using the median permutation test. **c,d.** Surviving fraction data from two independently-derived isogenic PTEN mutant/wild-type models transfected with siRNA targeting TTK. Surviving fractions were determined seven days after transfection. **p*<0.05 (Student's t test) **e,f.** Surviving fraction data from two independently-derived isogenic PTEN mutant/wild-type models exposed to two chemically distinct TTK inhibitors (AZ3146 or CCT132774). Surviving fraction was determined after seven days exposure to inhibitor. In each case the two-way ANOVA statistic of differences in survival curves is p<0.0001).

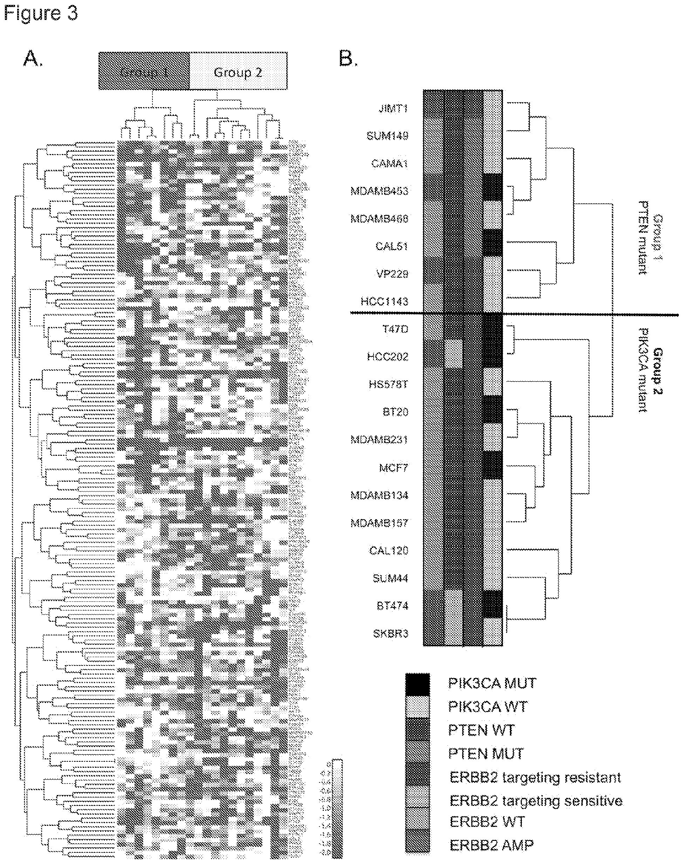

**Figure 3****. Hierarchical clustering of breast tumour cell lines according to functional viability profiles. a.** Heat map of breast tumour cell line functional viability data. Z score data from siRNAs that caused Z<-2 in two or more cell lines was used. pvclust approximately unbiased (au) p values are 0.92 and 0.91 for Group 1 and 2 respectively. **b.** Summary of hierarchical clustering. Group 1 was significantly enriched with *PTEN* mutant breast tumour cell lines (*p* = 0.0016), whereas Group 2 was significantly enriched with *PIK3CA* mutant breast tumour cell lines (*p* = 0.038). *p* values were calculated using a Chi-squared test.

### Detailed Description

### Inhibitors

Compounds which may be employed or screened for use as mitotic kinase inhibitors in accordance with the present invention for treating PTEN mutated or deficient cancer. Some inhibitors of these polypeptides are known and further examples may be found by the application of screening technologies to these targets.

### Small molecule inhibitors

By way of example, known inhibitors of TTK include relatively potent and orally bioavailable small molecules (PMID: 20383151) (PMID: 21159646). Primarily, the utility of TTK inhibitors has been proposed to be selective in tumors defined by high levels of chromosome instability (CIN) (PMID: 16921376) and have been shown to be effective in numerous cancer types (PMID: 21159646) As other spindle assembly check point (SAC) components have been found to be upregulated in a wide variety of tumors, it has been suggested that these cancers have become addicted to the SAC function and the mitotic check point is required to sustain their proliferation in the presence of aneuploidy (PMID: 17439397, PMID: 19269363,PMID: 18469805, PMID: 18383837). Further TTK inhibitors are disclosed in WO2010/007756, US2011/0002923 and US2003/0045491. These may be suitable for use in accordance with the present invention even though the respective applications do not disclose the use of these TTK inhibitors in the treatment of PTEN mutated or deficient cancers. The inhibitors include compounds AZ3146 or CCT132774 used in the examples below.

However, further mitotic kinase inhibitors have be identified by the use of high throughput screening strategies.

The present invention also extends to the use of small molecule inhibitors found in the screening disclosed herein and to Derivatives which are compounds of similar structure and functionality to the compounds found in the high throughput screen, but with one or more modifications, are expected to have similar physiological effects to these compounds and could therefore also be of use in the treatment of PTEN mutated or deficient cancer. The screening methods of the invention may be used to screen libraries of such derivatives to optimise their activity, if necessary.

Derivatives may be designed, based on a lead compound, by modifying one or more substituents or functional groups compared to the lead compound, for example by replacing these with alternative substituents or groups which are expected to have the same or improved physiological effect. The use of derivatives having such modifications is well known to those in the art.

### Antibodies

Antibodies may be employed in the present invention as an example of a class of inhibitor useful for treating PTEN mutated or deficient cancer, and more particularly as mitotic kinase inhibitors such as inhibitors of TTK protein kinase. They may also be used in the methods disclosed herein for assessing an individual having cancer or predicting the response of an individual having cancer, in particular for determining whether the individual has PTEN mutated or deficient cancer that might be treatable according to the present invention.

As used herein, the term "antibody" includes an immunoglobulin whether natural or partly or wholly synthetically produced. The term also covers any polypeptide or protein comprising an antibody binding domain. Antibody fragments which comprise an antigen binding domain are such as Fab, scFv, Fv, dAb, Fd; and diabodies. It is possible to take monoclonal and other antibodies and use techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP 0 184 187 A, GB 2,188,638 A or EP 0 239 400 A.

Antibodies can be modified in a number of ways and the term "antibody molecule" should be construed as covering any specific binding member or substance having an antibody antigen-binding domain with the required specificity. Thus, this term covers antibody fragments and derivatives, including any polypeptide comprising an immunoglobulin binding domain, whether natural or wholly or partially synthetic. Chimeric molecules comprising an immunoglobulin binding domain, or equivalent, fused to another polypeptide are therefore included. Cloning and expression of chimeric antibodies are described in EP 0 120 694 A and EP 0 125 023 A.

It has been shown that fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment (Ward, E.S. et al., Nature 341, 544-546 (1989)) which consists of a VH domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al, Science, 242; 423-426, 1988; Huston et al, PNAS USA, 85: 5879-5883, 1988); (viii) bispecific single chain Fv dimers (WO 93/11161) and (ix) "diabodies", multivalent or multispecific fragments constructed by gene fusion (WO 94/13804; Holliger et al, P.N.A.S. USA, 90: 6444-6448, 1993); (x) immunoadhesins (WO 98/50431). Fv, scFv or diabody molecules may be stabilised by the incorporation of disulphide bridges linking the VH and VL domains (Reiter et al, Nature Biotech, 14: 1239-1245, 1996). Minibodies comprising a scFv joined to a CH3 domain may also be made (Hu et al, Cancer Res., 56: 3055-3061, 1996).

Preferred antibodies used in accordance with the present invention are isolated, in the sense of being free from contaminants such as antibodies able to bind other polypeptides and/or free of serum components. Monoclonal antibodies are preferred for some purposes, though polyclonal antibodies are within the scope of the present invention.

The reactivities of antibodies on a sample may be determined by any appropriate means. Tagging with individual reporter molecules is one possibility. The reporter molecules may directly or indirectly generate detectable, and preferably measurable, signals. The linkage of reporter molecules may be directly or indirectly, covalently, e.g. via a peptide bond or non-covalently. Linkage via a peptide bond may be as a result of recombinant expression of a gene fusion encoding antibody and reporter molecule. One favoured mode is by covalent linkage of each antibody with an individual fluorochrome, phosphor or laser exciting dye with spectrally isolated absorption or emission characteristics. Suitable fluorochromes include fluorescein, rhodamine, phycoerythrin and Texas Red. Suitable chromogenic dyes include diaminobenzidine.

Other reporters include macromolecular colloidal particles or particulate material such as latex beads that are coloured, magnetic or paramagnetic, and biologically or chemically active agents that can directly or indirectly cause detectable signals to be visually observed, electronically detected or otherwise recorded. These molecules may be enzymes which catalyse reactions that develop or change colours or cause changes in electrical properties, for example. They may be molecularly excitable, such that electronic transitions between energy states result in characteristic spectral absorptions or emissions. They may include chemical entities used in conjunction with biosensors. Biotin/avidin or biotin/streptavidin and alkaline phosphatase detection systems may be employed.

Antibodies according to the present invention may be used in screening for the presence of a polypeptide, for example in a test sample containing cells or cell lysate as discussed, and may be used in purifying and/or isolating a polypeptide according to the present invention, for instance following production of the polypeptide by expression from encoding nucleic acid. Antibodies may modulate the activity of the polypeptide to which they bind and so, if that polypeptide has a deleterious effect in an individual, may be useful in a therapeutic context (which may include prophylaxis).

### Peptide fragments

Another class of inhibitors useful for treating PTEN mutated or deficient cancer includes peptide fragments that interfere with the activity of a mitotic kinase such as TTK protein kinase. Peptide fragments may be generated wholly or partly by chemical synthesis that block the catalytic sites of the kinase. Peptide fragments can be readily prepared according to well-established, standard liquid or, preferably, solid-phase peptide synthesis methods, general descriptions of which are broadly available (see, for example, in J.M. Stewart and J.D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984); and Applied Biosystems 430A Users Manual, ABI Inc., Foster City, California), or they may be prepared in solution, by the liquid phase method or by any combination of solid-phase, liquid phase and solution chemistry, e.g. by first completing the respective peptide portion and then, if desired and appropriate, after removal of any protecting groups being present, by introduction of the residue X by reaction of the respective carbonic or sulfonic acid or a reactive derivative thereof.

Other candidate compounds for inhibiting a mitotic kinase may be based on modelling the 3-dimensional structure of these enzymes and using rational drug design to provide candidate compounds with particular molecular shape, size and charge characteristics. A candidate inhibitor, for example, may be a "functional analogue" of a peptide fragment or other compound which inhibits the component. A functional analogue has the same functional activity as the peptide or other compound in question. Examples of such analogues include chemical compounds which are modelled to resemble the three dimensional structure of the component in an area which contacts another component, and in particular the arrangement of the key amino acid residues as they appear.

### Nucleic acid inhibitors

Another class of inhibitors useful for treatment of PTEN mutated or deficient cancer includes nucleic acid inhibitors of TTK protein kinase
, or the complements thereof, which inhibit activity or function by down-regulating production of active polypeptide. This can be monitored using conventional methods well known in the art, for example by screening using real time PCR as described in the examples.

Expression of mitotic kinases may be inhibited using anti-sense or RNAi technology. The use of these approaches to down-regulate gene expression is now well-established in the art.

Anti-sense oligonucleotides may be designed to hybridise to the complementary sequence of nucleic acid, pre-mRNA or mature mRNA, interfering with the production of the base excision repair pathway component so that its expression is reduced or completely or substantially completely prevented. In addition to targeting coding sequence, anti-sense techniques may be used to target control sequences of a gene, e.g. in the 5' flanking sequence, whereby the anti-sense oligonucleotides can interfere with expression control sequences. The construction of anti-sense sequences and their use is described for example in Peyman & Ulman, Chemical Reviews, 90:543-584, 1990 and Crooke, Ann. Rev. Pharmacol. Toxicol., 32:329-376, 1992.

Oligonucleotides may be generated *in vitro* or *ex vivo* for administration or anti-sense RNA may be generated *in vivo* within cells in which down-regulation is desired. Thus, double-stranded DNA may be placed under the control of a promoter in a "reverse orientation" such that transcription of the anti-sense strand of the DNA yields RNA which is complementary to normal mRNA transcribed from the sense strand of the target gene. The complementary anti-sense RNA sequence is thought then to bind with mRNA to form a duplex, inhibiting translation of the endogenous mRNA from the target gene into protein. Whether or not this is the actual mode of action is still uncertain. However, it is established fact that the technique works.

The complete sequence corresponding to the coding sequence in reverse orientation need not be used. For example fragments of sufficient length may be used. It is a routine matter for the person skilled in the art to screen fragments of various sizes and from various parts of the coding or flanking sequences of a gene to optimise the level of anti-sense inhibition. It may be advantageous to include the initiating methionine ATG codon, and perhaps one or more nucleotides upstream of the initiating codon. A suitable fragment may have about 14-23 nucleotides, e.g., about 15, 16 or 17 nucleotides.

An alternative to anti-sense is to use a copy of all or part of the target gene inserted in sense, that is the same, orientation as the target gene, to achieve reduction in expression of the target gene by co-suppression (Angell & Baulcombe, The EMBO Journal 16(12):3675-3684, 1997 and Voinnet & Baulcombe, Nature, 389: 553, 1997). Double stranded RNA (dsRNA) has been found to be even more effective in gene silencing than both sense or antisense strands alone (Fire et al, Nature 391, 806-811, 1998). dsRNA mediated silencing is gene specific and is often termed RNA interference (RNAi). Methods relating to the use of RNAi to silence genes in *C. elegans,* Drosophila, plants, and mammals are known in the art (Fire, Trends Genet., 15: 358-363, 19999; Sharp, RNA interference, Genes Dev. 15: 485-490 2001; Hammond et al., Nature Rev. Genet. 2: 110-1119, 2001; Tuschl, Chem. Biochem. 2: 239-245, 2001; Hamilton et al., Science 286: 950-952, 1999; Hammond, et al., Nature 404: 293-296, 2000; Zamore et al., Cell, 101: 25-33, 2000; Bernstein, Nature, 409: 363-366, 2001; Elbashir et al, Genes Dev., 15: 188-200, 2001; WO01/29058; WO99/32619, and Elbashir et al, Nature, 411: 494-498, 2001).

RNA interference is a two-step process. First, dsRNA is cleaved within the cell to yield short interfering RNAs (siRNAs) of about 21-23nt length with 5' terminal phosphate and 3' short overhangs (∼2nt). The siRNAs target the corresponding mRNA sequence specifically for destruction (Zamore, Nature Structural Biology, 8, 9, 746-750, 2001.

RNAi may also be efficiently induced using chemically synthesized siRNA duplexes of the same structure with 3'-overhang ends (Zamore et al, Cell, 101: 25-33, 2000). Synthetic siRNA duplexes have been shown to specifically suppress expression of endogenous and heterologeous genes in a wide range of mammalian cell lines (Elbashir et al, Nature, 411: 494-498, 2001).

Another possibility is that nucleic acid is used which on transcription produces a ribozyme, able to cut nucleic acid at a specific site and therefore also useful in influencing gene expression, e.g., see Kashani-Sabet & Scanlon, Cancer Gene Therapy, 2(3): 213-223, 1995 and Mercola & Cohen, Cancer Gene Therapy, 2(1): 47-59, 1995.

Small RNA molecules may be employed to regulate gene expression. These include targeted degradation of mRNAs by small interfering RNAs (siRNAs), post transcriptional gene silencing (PTGs), developmentally regulated sequence-specific translational repression of mRNA by micro-RNAs (miRNAs) and targeted transcriptional gene silencing.

A role for the RNAi machinery and small RNAs in targeting of heterochromatin complexes and epigenetic gene silencing at specific chromosomal loci has also been demonstrated. Double-stranded RNA (dsRNA)-dependent post transcriptional silencing, also known as RNA interference (RNAi), is a phenomenon in which dsRNA complexes can target specific genes of homology for silencing in a short period of time. It acts as a signal to promote degradation of mRNA with sequence identity. A 20-nt siRNA is generally long enough to induce gene-specific silencing, but short enough to evade host response. The decrease in expression of targeted gene products can be extensive with 90% silencing induced by a few molecules of siRNA.

In the art, these RNA sequences are termed "short or small interfering RNAs" (siRNAs) or "microRNAs" (miRNAs) depending on their origin. Both types of sequence may be used to down-regulate gene expression by binding to complimentary RNAs and either triggering mRNA elimination (RNAi) or arresting mRNA translation into protein. siRNA are derived by processing of long double stranded RNAs and when found in nature are typically of exogenous origin. Micro-interfering RNAs (miRNA) are endogenously encoded small non-coding RNAs, derived by processing of short hairpins. Both siRNA and miRNA can inhibit the translation of mRNAs bearing partially complimentary target sequences without RNA cleavage and degrade mRNAs bearing fully complementary sequences.

The siRNA ligands are typically double stranded and, in order to optimise the effectiveness of RNA mediated down-regulation of the function of a target gene, it is preferred that the length of the siRNA molecule is chosen to ensure correct recognition of the siRNA by the RISC complex that mediates the recognition by the siRNA of the mRNA target and so that the siRNA is short enough to reduce a host response.

miRNA ligands are typically single stranded and have regions that are partially complementary enabling the ligands to form a hairpin. miRNAs are RNA genes which are transcribed from DNA, but are not translated into protein. A DNA sequence that codes for a miRNA gene is longer than the miRNA. This DNA sequence includes the miRNA sequence and an approximate reverse complement. When this DNA sequence is transcribed into a single-stranded RNA molecule, the miRNA sequence and its reverse-complement base pair to form a partially double stranded RNA segment. The design of microRNA sequences is discussed in John et al, PLoS Biology, 11(2), 1862-1879, 2004.

Typically, the RNA ligands intended to mimic the effects of siRNA or miRNA have between 10 and 40 ribonucleotides (or synthetic analogues thereof), more preferably between 17 and 30 ribonucleotides, more preferably between 19 and 25 ribonucleotides and most preferably between 21 and 23 ribonucleotides. In some embodiments of the invention employing double-stranded siRNA, the molecule may have symmetric 3' overhangs, e.g. of one or two (ribo)nucleotides, typically a UU of dTdT 3' overhang. Based on the disclosure provided herein, the skilled person can readily design suitable siRNA and miRNA sequences, for example using resources such as Ambion's siRNA finder, see http://www.ambion.com/techlib/misc/siRNA_finder.html. siRNA and miRNA sequences can be synthetically produced and added exogenously to cause gene downregulation or produced using expression systems (e.g. vectors). In a preferred embodiment the siRNA is synthesized synthetically.

Longer double stranded RNAs may be processed in the cell to produce siRNAs (e.g. see Myers, Nature Biotechnology, 21: 324-328, 2003). The longer dsRNA molecule may have symmetric 3' or 5' overhangs, e.g. of one or two (ribo)nucleotides, or may have blunt ends. The longer dsRNA molecules may be 25 nucleotides or longer. Preferably, the longer dsRNA molecules are between 25 and 30 nucleotides long. More preferably, the longer dsRNA molecules are between 25 and 27 nucleotides long. Most preferably, the longer dsRNA molecules are 27 nucleotides in length. dsRNAs 30 nucleotides or more in length may be expressed using the vector pDECAP (Shinagawa et al., Genes and Dev., 17: 1340-5, 2003).

Another alternative is the expression of a short hairpin RNA molecule (shRNA) in the cell. shRNAs are more stable than synthetic siRNAs. A shRNA consists of short inverted repeats separated by a small loop sequence. One inverted repeat is complimentary to the gene target. In the cell the shRNA is processed by DICER into a siRNA which degrades the target gene mRNA and suppresses expression. In a preferred embodiment the shRNA is produced endogenously (within a cell) by transcription from a vector. shRNAs may be produced within a cell by transfecting the cell with a vector encoding the shRNA sequence under control of a RNA polymerase III promoter such as the human H1 or 7SK promoter or a RNA polymerase II promoter. Alternatively, the shRNA may be synthesised exogenously (in vitro) by transcription from a vector. The shRNA may then be introduced directly into the cell. Preferably, the shRNA sequence is between 40 and 100 bases in length, more preferably between 40 and 70 bases in length. The stem of the hairpin is preferably between 19 and 30 base pairs in length. The stem may contain G-U pairings to stabilise the hairpin structure.

In one embodiment, the siRNA, longer dsRNA or miRNA is produced endogenously (within a cell) by transcription from a vector. The vector may be introduced into the cell in any of the ways known in the art. Optionally, expression of the RNA sequence can be regulated using a tissue specific promoter. In a further embodiment, the siRNA, longer dsRNA or miRNA is produced exogenously (*in vitro*) by transcription from a vector.

Alternatively, siRNA molecules may be synthesized using standard solid or solution phase synthesis techniques, which are known in the art. Linkages between nucleotides may be phosphodiester bonds or alternatives, e.g., linking groups of the formula P(O)S, (thioate); P(S)S, (dithioate); P(O)NR'2; P(O)R'; P(O)OR6; CO; or CONR'2 wherein R is H (or a salt) or alkyl (1-12C) and R6 is alkyl (1-9C) is joined to adjacent nucleotides through-O-or-S-.

Modified nucleotide bases can be used in addition to the naturally occurring bases, and may confer advantageous properties on siRNA molecules containing them.

For example, modified bases may increase the stability of the siRNA molecule, thereby reducing the amount required for silencing. The provision of modified bases may also provide siRNA molecules, which are more, or less, stable than unmodified siRNA.

The term 'modified nucleotide base' encompasses nucleotides with a covalently modified base and/or sugar. For example, modified nucleotides include nucleotides having sugars, which are covalently attached to low molecular weight organic groups other than a hydroxyl group at the 3'position and other than a phosphate group at the 5'position. Thus modified nucleotides may also include 2'substituted sugars such as 2'-O-methyl- ; 2-0-alkyl ; 2-0-allyl ; 2'-S-alkyl; 2'-S-allyl; 2'-fluoro- ; 2'-halo or 2; azido-ribose, carbocyclic sugar analogues α-anomeric sugars; epimeric sugars such as arabinose, xyloses or lyxoses, pyranose sugars, furanose sugars and sedoheptulose.

Modified nucleotides are known in the art and include alkylated purines and pyrimidines, acylated purines and pyrimidines, and other heterocycles. These classes of pyrimidines and purines are known in the art and include pseudoisocytosine, N4,N4-ethanocytosine, 8-hydroxy-N6-methyladenine, 4-acetylcytosine,5-(carboxyhydroxylmethyl) uracil, 5 fluorouracil, 5-bromouracil, 5-carboxymethylaminomethyl-2-thiouracil, 5-carboxymethylaminomethyl uracil, dihydrouracil, inosine, N6-isopentyl-adenine, 1-methyladenine, 1-methylpseudouracil, 1-methylguanine, 2,2-dimethylguanine, 2methyladenine, 2-methylguanine, 3-methylcytosine, 5-methylcytosine, N6-methyladenine, 7-methylguanine, 5-methylaminomethyl uracil, 5-methoxy amino methyl-2-thiouracil, -D-mannosylqueosine, 5-methoxycarbonylmethyluracil, 5methoxyuracil, 2 methylthio-N6-isopentenyladenine, uracil-5-oxyacetic acid methyl ester, psueouracil, 2-thiocytosine, 5-methyl-2 thiouracil, 2-thiouracil, 4-thiouracil, 5methyluracil, N-uracil-5-oxyacetic acid methylester, uracil 5-oxyacetic acid, queosine, 2-thiocytosine, 5-propyluracil, 5-propylcytosine, 5-ethyluracil, 5ethylcytosine, 5-butyluracil, 5-pentyluracil, 5-pentylcytosine, and 2,6,diaminopurine, methylpsuedouracil, 1-methylguanine, 1-methylcytosine.

### Methods of screening

In some aspects, the present invention is concerned with methods of screening candidate compounds to determine whether one or more candidate agents are likely to be useful for the treatment of PTEN mutated or deficient cancer. As described herein, there are three preferred general approaches that may be used for these methods of screening, either alone or in any combination or order.

In the screening methods of the present invention, it may be advantageous to use a PTEN mutant cell line to test the effectiveness of a candidate TTK protein kinase inhibitor. A full list of PTEN mutant cell lines is available for retrieved from the COSMIC database:
http://www.sanger.ac.uk/perl/genetics/CGP/cosmic?action=mutations &ln=PTEN&start=1&end=404&coords=AA:AA&page=1)

In a first approach, a method of screening may involve using cell lines to determine whether a candidate agent is synthetically lethal in a first cell line which is PTEN mutated or deficient. This method preferably also uses a second cell line that is PTEN proficient as a control and candidate agents are selected which are synthetically lethal in the first cell line and which preferably do not cause any substantial amount cell death in the second cell line and/or normal cells. Thus, in this embodiment of the invention exploits synthetic lethality in cancer cells. Two mutations are synthetically lethal if cells with either of the single mutations are viable, but cells with both mutations are inviable. Identifying synthetic lethal combinations therefore allows a distinct approach to identifying therapeutic targets that allows selective killing of tumour cells. Preferably, the method is carried out using cancer cell lines, e.g. mammalian or human cancer cell lines, and more specifically PTEN mutated or deficient cancer cell lines.

One preferred way of initially identifying synthetic lethal interactions involves the use of RNAi screens. Synthetic lethality describes the scenario in which two normally non-essential genes become essential when both are lost, or inhibited. Targeting a gene that is synthetically lethal with a cancer specific mutation should selectively kill tumour cells while sparing normal cells. One of the major advantages of this approach is the ability to target cancer cells containing loss-of-function mutations, that is, mutations in tumour suppressor genes. Previously, it has been difficult to devise therapeutic strategies to target these mutations as recapitulating tumour suppressor function is technically difficult. Most pharmacological agents inhibit rather than activate protein function and therefore cannot be used to target loss-of-function alterations in tumours. Identification of synthetic lethal relationships with tumour suppressor genes could allow cells that contain the tumour suppressor mutations to be selectively killed.

The use of synthetic lethality to target cancer-specific mutations has been demonstrated by the selective killing of PTEN mutated or deficient cancer cells using mitotic kinase inhibitors. These inhibitors showed profound selectivity, killing cells with PTEN mutations or deficiency, while normal cells were unaffected. However, with the advent of high-throughput RNAi screens it is now possible, in principle, to perform large-scale synthetic-lethal gene identification in mammalian cells, as is routinely done in yeast. Screening deletion mutants that have defects in cell-cycle checkpoint or DNA repair mechanisms in yeast has yielded synthetically lethal genes and small-molecule inhibitors. Using mammalian isogenic-paired cell lines that differ in a single genetic target, RNAi can be used to identify drug targets that when inhibited will result in the selective death of tumour cells.

Chemical screens have been performed previously on isogenic cancer cell lines for synthetic lethal interactions. However, such approaches have the significant disadvantage of having to identify the cellular targets of an active small molecule. This can be achieved by illustrating the affinity of a small molecule for a particular protein, but this is time-consuming and suffers the limitation that irrelevant proteins will bind in addition to the target. A variation on the synthetic lethality theme is to use compounds that inhibit a cancer-specific target and then screen RNAi libraries to identify targets that selectively kill the cells treated with this compound.

The present invention also includes methods of screening that employ mitotic kinases as protein targets for the screening of candidate compounds to find mitotic kinase inhibitors. Accordingly, methods of screening may be carried out for identifying candidate agents that are capable of inhibiting
TTK protein kinase for subsequent use of development as agents for the treatment of PTEN mutated or deficient cancer. Conveniently, this may be done in an assay buffer to help the components of the assay interact, and in a multiple well format to test a plurality of candidate agents.

The activity of a mitotic kinase can then be determined in the presence and absence of the one or more candidate compounds to determine whether a given candidate is an inhibitor of a mitotic kinase.

By way of example, the candidate agent may be a known inhibitor of one of the protein targets disclosed herein, an antibody, a peptide, a nucleic acid molecule or an organic or inorganic compound, e.g. molecular weight of less than 100 Da. In some instances the use of candidate agents that are compounds is preferred. However, for any type of candidate agent, combinatorial library technology provides an efficient way of testing a potentially vast number of different substances for ability to modulate activity of a target protein. Such libraries and their use are known in the art. The present invention also specifically envisages screening candidate agents known for the treatment of other conditions, and especially other forms of cancer. This has the advantage that the patient or disease profile of known therapeutic agents might be expanded or modified using the screening techniques disclosed herein, or for therapeutic agents in development, patient or disease profiles established that are relevant for the treatment of PTEN mutated or deficient cancer.

Following identification of a candidate agent for further investigation, the agent in question may be tested to determine whether it is not lethal to normal cells or otherwise is suited to therapeutic use. Following these studies, the agent may be manufactured and/or used in the preparation of a medicament, pharmaceutical composition or dosage form.

The development of lead agents or compounds from an initial hit in screening assays might be desirable where the agent in question is difficult or expensive to synthesise or where it is unsuitable for a particular method of administration, e.g. peptides are unsuitable active agents for oral compositions as they tend to be quickly degraded by proteases in the alimentary canal. Mimetic design, synthesis and testing is generally used to avoid randomly screening large number of molecules for a target property.

There are several steps commonly taken in the design of a mimetic from a compound having a given target property. Firstly, the particular parts of the compound that are critical and/or important in determining the target property are determined. In the case of a peptide, this can be done by systematically varying the amino acid residues in the peptide, e.g. by substituting each residue in turn. These parts or residues constituting the active region of the compound are known as its "pharmacophore".

Once the pharmacophore has been found, its structure is modelled to according its physical properties, e.g. stereochemistry, bonding, size and/or charge, using data from a range of sources, e.g. spectroscopic techniques, X-ray diffraction data and NMR. Computational analysis, similarity mapping (which models the charge and/or volume of a pharmacophore, rather than the bonding between atoms) and other techniques can be used in this modelling process. In a variant of this approach, the three-dimensional structure of the ligand and its binding partner are modelled. This can be especially useful where the ligand and/or binding partner change conformation on binding, allowing the model to take account of this in the design of the mimetic.

A template molecule is then selected onto which chemical groups which mimic the pharmacophore can be grafted. The template molecule and the chemical groups grafted on to it can conveniently be selected so that the mimetic is easy to synthesise, is likely to be pharmacologically acceptable, and does not degrade *in vivo,* while retaining the biological activity of the lead compound. The mimetics found by this approach can then be screened to see whether they have the target property, or to what extent they exhibit it. Further optimisation or modification can then be carried out to arrive at one or more final mimetics for *in vivo* or clinical testing.

### Treatment of cancer

The present invention provides methods and medical uses for the treatment of PTEN mutated or deficient cancer. A cancer may be identified as PTEN mutated or deficient cancer by testing a sample of cancer cells from an individual, for example to determine whether the PTEN protein contains one or more mutations or to determine the expression of the PTEN gene to evaluate whether expression of the protein is absent or at a reduced level compared to normal. It is known that genetic inactivation of PTEN occurs in glioblastoma, endometrial cancer and prostate cancer and reduced expression is found in many other tumor types that include lung and breast cancer. Examples of known PTEN mutated or deficient cancers that are treatable in accordance with the present invention include cancers affecting the autonomic ganglia, biliary tract, bone, breast, CNS, cervix, endometrium, eye, haematopoietic and lymphoid tissue, kidney, large intestine, liver, lung, meninges, oesophagus, ovary, pancreas, prostate, salivary gland, skin, soft tissue, stomach, testis, thyroid, upper aerodigestive tract, urinary tract, or vulva. Cancer associated mutations in PTEN occur across the entire coding sequence of the gene and include both premature truncating mutations and single amino acid substitutions in both the catalytic and non-catalytic domains of the protein. The frequency of monoallelic mutations in PTEN has been estimated at 50%-80% in sporadic tumors (including endometrial carcinoma, glioblastoma, and prostate cancer) and at 30%-50% in breast, colon, and lung tumors. Complete loss of *PTEN* is observed at highest frequencies in endometrial cancer and glioblastoma and is generally associated with advanced cancers and metastases. Common tumor associated PTEN mutations include c.388C>G, c.697C>T, c.388C>T, c.800delA, c.968delA, c.517C>T, c.968_969insA, c.518G>A, c.955_958delACTT, c.1003C>T, c.950_953delTACT.

The sample may be of normal cells from the individual where the individual has a mutation in the PTEN gene or the sample may be of cancer cells, e.g. where the cells forming a tumour contain one or more PTEN mutations. Activity may be determined relative to a control, for example in the case of defects in cancer cells, relative to non-cancerous cells, preferably from the same tissue.

The activity of the PTEN may be determined by using techniques well known in the art such as Western blot analysis, immunohistology, chromosomal abnormalities, enzymatic or DNA binding assays and plasmid-based assays.

The determination of PTEN gene expression may involve determining the presence or amount of PTEN mRNA in a sample. Methods for doing this are well known to the skilled person. By way of example, they include determining the presence of PTEN mRNA (i) using a labelled probe that is capable of hybridising to the PTEN nucleic acid; and/or (ii) using PCR involving one or more primers based on a PTEN nucleic acid sequence to determine whether the PTEN transcript is present in a sample. The probe may also be immobilised as a sequence included in a microarray.

Preferably, detecting PTEN mRNA is carried out by extracting RNA from a sample of the tumour and measuring PTEN expression specifically using quantitative real time RT-PCR. Alternatively or additionally, the expression of PTEN could be assessed using RNA extracted from a tumour sample using microarray analysis, which measures the levels of mRNA for a group of genes using a plurality of probes immobilised on a substrate to form the array.

Alternatively or additionally, the present invention the determination of whether a patient has a PTEN mutated or deficient cancer can be carried out by analysis of PTEN protein expression, for example to examining whether reduced levels of PTEN protein are expressed or whether the PTEN protein contains one or more mutations.

Preferably, the presence or amount of PTEN protein may be determined using a binding agent capable of specifically binding to the PTEN protein, or fragments thereof. A preferred type of PTEN protein binding agent is an antibody capable of specifically binding the PTEN or fragment thereof. The antibody may be labelled to enable it to be detected or capable of detection following reaction with one or more further species, for example using a secondary antibody that is labelled or capable of producing a detectable result, e.g. in an ELISA type assay. As an alternative a labelled binding agent may be employed in a western blot to detect PTEN protein.

Alternatively, or additionally, the method for determining the presence of PTEN protein may be carried out on tumour samples, for example using immunohistochemical (IHC) analysis. IHC analysis can be carried out using paraffin fixed samples or frozen tissue samples, and generally involves staining the samples to highlight the presence and location of PTEN protein.

### Pharmaceutical compositions

The active agents disclosed herein for the treatment of PTEN mutated or deficient cancer may be administered alone, but it is generally preferable to provide them in pharmaceutical compositions that additionally comprise with one or more pharmaceutically acceptable carriers, adjuvants, excipients, diluents, fillers, buffers, stabilisers, preservatives, lubricants, or other materials well known to those skilled in the art and optionally other therapeutic or prophylactic agents. Examples of components of pharmaceutical compositions are provided in Remington's Pharmaceutical Sciences, 20th Edition, 2000, pub. Lippincott, Williams & Wilkins.

Examples of small molecule therapeutics useful for treating PTEN mutated or deficient cancer include: BEZ235, Olaparib and GDC0941.

These compounds or derivatives of them may be used in the present invention for the treatment of PTEN mutated or deficient cancer. As used herein "derivatives" of the therapeutic agents includes salts, coordination complexes, esters such as in vivo hydrolysable esters, free acids or bases, hydrates, prodrugs or lipids, coupling partners.

Salts of the compounds of the invention are preferably physiologically well tolerated and non toxic. Many examples of salts are known to those skilled in the art. Compounds having acidic groups, such as phosphates or sulfates, can form salts with alkaline or alkaline earth metals such as Na, K, Mg and Ca, and with organic amines such as triethylamine and Tris (2-hydroxyethyl)amine. Salts can be formed between compounds with basic groups, e.g., amines, with inorganic acids such as hydrochloric acid, phosphoric acid or sulfuric acid, or organic acids such as acetic acid, citric acid, benzoic acid, fumaric acid, or tartaric acid. Compounds having both acidic and basic groups can form internal salts.

Esters can be formed between hydroxyl or carboxylic acid groups present in the compound and an appropriate carboxylic acid or alcohol reaction partner, using techniques well known in the art.

Derivatives which as prodrugs of the compounds are convertible in vivo or in vitro into one of the parent compounds. Typically, at least one of the biological activities of compound will be reduced in the prodrug form of the compound, and can be activated by conversion of the prodrug to release the compound or a metabolite of it.

Other derivatives include coupling partners of the compounds in which the compounds is linked to a coupling partner, e.g. by being chemically coupled to the compound or physically associated with it. Examples of coupling partners include a label or reporter molecule, a supporting substrate, a carrier or transport molecule, an effector, a drug, an antibody or an inhibitor. Coupling partners can be covalently linked to compounds of the invention via an appropriate functional group on the compound such as a hydroxyl group, a carboxyl group or an amino group. Other derivatives include formulating the compounds with liposomes.

The term "pharmaceutically acceptable" as used herein includes compounds, materials, compositions, and/or dosage forms which are, within the scope of sound medical judgement, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

The active agents disclosed herein for the treatment of PTEN mutated or deficient cancer according to the present invention are preferably for administration to an individual in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc., is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 20th Edition, 2000, Lippincott, Williams & Wilkins. A composition may be administered alone or in combination with other treatments, either simultaneously or sequentially, dependent upon the condition to be treated.

The formulations may conveniently be presented in unit dosage form and may be prepared by any methods well known in the art of pharmacy. Such methods include the step of bringing the active compound into association with a carrier, which may constitute one or more accessory ingredients. In general, the formulations are prepared by uniformly and intimately bringing into association the active compound with liquid carriers or finely divided solid carriers or both, and then if necessary shaping the product.

The agents disclosed herein for the treatment of PTEN mutated or deficient cancer may be administered to a subject by any convenient route of administration, whether systemically/ peripherally or at the site of desired action, including but not limited to, oral (e.g. by ingestion); topical (including e.g. transdermal, intranasal, ocular, buccal, and sublingual); pulmonary (e.g. by inhalation or insufflation therapy using, e.g. an aerosol, e.g. through mouth or nose); rectal; vaginal; parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; by implant of a depot, for example, subcutaneously or intramuscularly.

Formulations suitable for oral administration (e.g., by ingestion) may be presented as discrete units such as capsules, cachets or tablets, each containing a predetermined amount of the active compound; as a powder or granules; as a solution or suspension in an aqueous or non-aqueous liquid; or as an oil-in-water liquid emulsion or a water-in-oil liquid emulsion; as a bolus; as an electuary; or as a paste.

Formulations suitable for parenteral administration (e.g., by injection, including cutaneous, subcutaneous, intramuscular, intravenous and intradermal), include aqueous and non-aqueous isotonic, pyrogen-free, sterile injection solutions which may contain anti-oxidants, buffers, preservatives, stabilisers, bacteriostats, and solutes which render the formulation isotonic with the blood of the intended recipient; and aqueous and non-aqueous sterile suspensions which may include suspending agents and thickening agents, and liposomes or other microparticulate systems which are designed to target the compound to blood components or one or more organs. Examples of suitable isotonic vehicles for use in such formulations include Sodium Chloride Injection, Ringer's Solution, or Lactated Ringer's Injection. Typically, the concentration of the active compound in the solution is from about 1 ng/ml to about 10 µg/ml, for example from about 10 ng/ml to about 1 µg/ml. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets. Formulations may be in the form of liposomes or other microparticulate systems which are designed to target the active compound to blood components or one or more organs.

Compositions comprising agents disclosed herein for the treatment PTEN mutated or deficient cancer may be used in the methods described herein in combination with standard chemotherapeutic regimes or in conjunction with radiotherapy. Examples of other chemotherapeutic agents include Amsacrine (Amsidine), Bleomycin, Busulfan, Capecitabine (Xeloda), Carboplatin, Carmustine (BCNU), Chlorambucil(Leukeran), Cisplatin, Cladribine(Leustat), Clofarabine (Evoltra), Crisantaspase (Erwinase), Cyclophosphamide, Cytarabine (ARA-C), Dacarbazine (DTIC), Dactinomycin (Actinomycin D),Daunorubicin, Docetaxel (Taxotere), Doxorubicin, Epirubicin, Etoposide (Vepesid, VP-16), Fludarabine (Fludara), Fluorouracil (5-FU), Gemcitabine (Gemzar), Hydroxyurea (Hydroxycarbamide, Hydrea) ,Idarubicin (Zavedos). Ifosfamide (Mitoxana), Irinotecan (CPT-11, Campto), Leucovorin (folinic acid), Liposomal doxorubicin (Caelyx, Myocet), Liposomal daunorubicin (DaunoXome®) Lomustine, Melphalan, Mercaptopurine, Mesna, Methotrexate, Mitomycin, Mitoxantrone, Oxaliplatin (Eloxatin), Paclitaxel (Taxol), Pemetrexed (Alimta), Pentostatin (Nipent), Procarbazine, Raltitrexed (Tomudex®), Streptozocin (Zanosar®), Tegafur-uracil (Uftoral), Temozolomide (Temodal), Teniposide (Vumon), Thiotepa, Tioguanine (6-TG) (Lanvis), Topotecan (Hycamtin), Treosulfan, Vinblastine (Velbe), Vincristine (Oncovin), Vindesine (Eldisine) and Vinorelbine (Navelbine).

Administration *in vivo* can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell being treated, and the subject being treated. Single or multiple administrations can be carried out with the dose level and pattern being selected by the treating physician.

In general, a suitable dose of the active compound is in the range of about 100 µg to about 250 mg per kilogram body weight of the subject per day. Where the active compound is a salt, an ester, prodrug, or the like, the amount administered is calculated on the basis of the parent compound, and so the actual weight to be used is increased proportionately.

### Experimental Examples

### METHODS

### Cell lines, compounds and siRNA

All cell lines were obtained from ATCC (USA) and maintained according to the supplier's instructions. Cell lines were grown and transfected with SMARTpool siRNAs using Dharmafect 3 (DF3), Dharmafect 4 (DF4) (Dharmacon), Oligofectamine, Lipofectamine 2000 or RNAiMAX (Invitrogen) transfection reagents. The siRNA library (siARRAY - targeting 714 known and putative human protein kinase genes) was obtained in nine 96 well plates from Dharmacon (USA). Each well in this library contained a SMARTpool of four distinct siRNA species targeting different sequences of the target transcript. Each plate was supplemented with siCONTROL (ten wells, Dharmacon (USA)).

### Antibodies

Antibodies targeting the following were used as per manufacturers instructions: ACTIN and C-MYC (Santa Cruz Biotech), TTK, ER, PR, ERBB2, CYCLIN D1, TFF1, FOXO1, C-JUN, and PTEN C-terminus (Cell Signalling, Danvers, USA). All secondary antibodies used for western blot analysis were HRP conjugated.

### RNA interference screening

We transfected cells with the SMARTpool library, where each well of the 96 wellplate contained a pool of four different siRNAs (a SMARTpool) targeting one gene. After five population doublings , cell viability in each well was estimated by use of a highly sensitive luminescent assay measuring cellular ATP levels (Cell Titre Glo, Promega). To identify loss of viability/failure to proliferate effects in each cell line, luminescence readings from each well were log transformed and then centred by the plate median, to account for plate-to-plate variation common in high-throughput screens. Well position effects were identified and eliminated and the quality of data from each plate estimated by calculating Z'-factors(24) based on positive (siRNA targeting PLK1) and negative (non-targeting siRNA) controls in each plate. The dynamic range of each screen was determined by calculating Z prime values(24); we use a threshold of Z prime >0.3 to define acceptable screens, based upon previous screens where Z prime >0.3 is predictive of reproducible data(25, 26).

To allow data to be compared between different cell lines, plate-centred data from each screen were standardised by the use of a Z score statistic, where Z=0 represents no effect on viability and negative Z scores represent loss of viability. Here, Z scores were calculated using the Median Absolute Deviation (MAD) of all effects in each cell line(5). In each screen the Z score data approximated a normal distribution allowing comparison of the individual siRNA effects across cell lines. siRNA screens were carried out in triplicate and comparison of Z score data from replica screens of each cell line showed the screening process to be highly robust, as demonstrated by Spearman's r2 values approaching 1 for all comparisons . Quality metrics for each screen were confirmed. Raw luminescence values and processed data are now deposited on the ROCK Breast Cancer Functional Genomics Database(23) (rock.icr.ac.uk) as a community resource. This rigorous quality control method reduced our cell line panel to 20 cell lines with robust viability profiles. Heatmaps of Z scores that separated different phenotypes as defined by a median difference permutation test were created to show the significant genes ranked row-wise according to their median difference and ordered column-wise by phenotype.

### Western blots

Protein lysates were prepared using RIPA lysis buffer (50 nM Tris pH 8.0, 150 mM NaCl, 0.1% SDS, 0.1% DOC, 1% TritonX-100, 50 mM NaF, 1 mM Na₃VO₄ and protease inhibitors). 100mg of total cell lysate was loaded onto prefabricated 4-12% Bis-Tris gels (Invitrogen), with full range rainbow molecular weight marker (GE Healthcare, UK) as a size reference, and resolved by SDS-PAGE electrophoresis. Proteins were transferred to nitrocellulose membrane (Bio-rad, USA), blocked and probed with primary antibody diluted 1 in 1000 in 5% milk overnight at 4°C. Secondary antibodies were diluted 1 in 5000 in 5% milk and incubated for one hour at room temperature. Protein bands were visualised using ECL (GE Healthcare, UK) and MR or XAR film (Kodak).

### Validation of gene silencing by siRNA

Validation of RNAi gene silencing was determined by western blotting and by viability assays of silencing effects with individual oligos. Cells were transfected with individual ERBB2, ESR1, PIK3CA or TTK siGenome oligos (Dharmacon). Protein lysates were collected 48 hours following transfection for western blot analysis. Cell viability was measured using CellTiter Glo (Promega, USA) after 5 populations doublings.

### Survival assays

For measurement of sensitivity to TTK inhibitor treatment, cells were plated in 96 well plates and exposed to the drug at the indicated concentrations. Cells were dosed at 24 and 96 hours. After 7 days, cell viability was measured using CellTiter Glo Luminescent Cell Viability Assay (Promega, USA). Surviving fractions were calculated and drug sensitivity curves plotted as previously described(27).

### Metaphase Spreads

After 24 hours of treatment with 2uM AZ3146, HCT116 PTEN wt or PTEN null cells were treated with colcemide (10ng/ml, Sigma) and MG132 (20uM, Sigma) for 1 hour. Cells were then lysed in hypotonic solution (0.03M Sodium Citrate) for 20 minutes at 37°C and fixed in methanol/acetic acid (3:1). Two or three drops of suspended cells were applied to glass slides and chromosomes were stained with DAPI.

### PTEN FISH

Cell pellets of CAl51 and Hec-1b were washed twice with PBS and fixated with methanol/acetic acid solution (3:1) onto slides. LSI PTEN (10q23, red/orange) /chromosome 10 centromere (CEP 10, green) Dual Colour Probe (Abbott Molecular, IL, US) was hybridised to representative slides of the cell lines according to the manufacturer's instructions. Signals were counted in 100 non-overlapping nuclei using the Leica TCS SP2 confocal microscope (Leica, Milton Keynes, UK).

### Transcript profiling

RNA was extracted from cell lines with Trizol and phenol/chloroform extraction followed by isopropanol precipitation. For each cell line, triplicate extractions and profiles were performed. Biotin-labelled cRNA was produced by means of a linear amplification kit (IL1791; Ambion, Austin, TX, http://www. ambion.com) using 250 ng of quality-checked total RNA as input. Chip hybridisations, washing, Cy3-streptavidin (Amersham Bio- sciences) staining, and scanning were performed on an Illumina BeadStation 500 (San Diego, http://www.illumina.com) platform using reagents and following protocols supplied by the manufacturer as previously described(28). cRNA
samples were hybridised on Illumina human-6 v2 BeadChips, covering approximately 47,000 RefSeq transcripts. The random distribution of large populations of oligonucleotide-coated beads across the available positions within the human-6 v2 chip enables, on average, 30 intensity measurements per RefSeq, yielding quantitative assessments of gene expression. All basic expression data analysis was carried out using the manufacturer's software BeadStudio 3.1. Illumina expression profiles were performed in triplicate, the raw data were then variance-stabilizing transformed and robust spine normalised using the *lumi* package in the Bioconductor software. Expression values for each sample were median scaled and the mean expression value was established over the three replicates. Genes with significant difference in expression between cell lines were identified by one-way analysis of variance (ANOVA).

### Array CGH (aCGH) analysis method

Genomic DNA was extracted from cell lines using the QIAamp DNA Blood Mini Kit (51104, Qiagen), according to manufacturer's instructions. Microarray-based CGH analysis was performed on an in-house 32K tiling path BAC array platform as previously described (29). For copy number correlations, the circular binary segmentation (CBS) ratios of BACs containing the gene of interest were used for copy number correlations, and copy number assigned as previously described (29). Briefly, AWS smoothed log2 ratio values <-0.12 were categorised as losses, those >0.12 as gains, and those in between as unchanged. Amplifications were defined as smoothed log2 ratio values >0.4 (29). Data processing and analysis were carried out in R 2.9.0 (http://www.r-project.org/) and BioConductor 1.5 (http://www.bioconductor.org/), making extensive use of modified versions of the packages aCGH, marray and aws in particular.

### Correlation of gene expression and aCGH data

aCGH and gene expression were compared by direct Pearson correlation of gene expression log intensity values with smoothed log ratio values for every probe in the gene expression data. Correlation p values were adjusted for multiple comparison testing using the local False Discovery Rate (FDR) method of Benjamini and Hochberg (30) as previously described (28). Mann Whitney U tests were preformed for each gene to compare gene expression values in groups defined as assigned as amplified or not, gained or not, lost or not and deleted or not using the thresholded aCGH calls. Wilcox test p-values were corrected for multiple comparison testing within contiguously altered regions. For each gene, cases in which genes were amplified, gained, lost or deleted were recorded along with the fold changes between samples carrying a copy number change and those which did not. Total counts of gains, losses, amplifications and deletions were also recorded.

*Correlation of siRNA Z score with gene expression and aCGH data* The correlation between siRNA Z score and normalised gene expression was examined for genes where siRNA caused significant loss of viability (Z<-2). Z score was compared to normalized gene expression using Pearson correlation coefficient. A gene was taken as being significantly correlated if the Pearson correlation coefficient was significantly different to the null hypothesis, the correlation was inverse, and the variation in gene expression between cells lines were significantly different as assessed by one-way ANOVA.

For aCGH overlay, Z scores for the common 714 genes in the gene expression and the kinase library were correlated directly using Pearson correlation and p-values adjusted for multiple comparison testing using the FDR (28). Gene expression values were subsequently compared in those cell lines that displayed Z score hits for each gene and those that did not. Fold changes between these groups for each gene were also calculated. Array CGH copy number changes for each gene were used to divide the cell lines into those carrying amplifications, gains, losses or deletions, from those which did not and Mann Whitney U tests were performed to compare gene expression between these groups for each gene. The numbers of cases harbouring amplifications, gains losses and deletions which also responded with Z score hits in the same cell line was also calculated.

### Clustering Methods

Supervised analysis was performed by calculating the absolute difference in the medians of the two groups, followed by estimating a p-value by permuting the labels to create a distribution of median differences against which to compare the actual effects. We used a significance cut-off of *p*=0.05 with no corrections for multiple comparisons. The combined gene expression, Z scores and aCGH matrices were subjected to hierarchichal clustering using a Pearson correlation distance measure and Wards clustering algorithm. Equivalent heatmaps were drawn which clustered the samples and the genes in the combined overlay using the corresponding Z scores. Heatmaps of gene expression and copy number changes were then displayed under the same dendrograms derived by clustering by Z score. Stability of the clustering was established by pvclust giving an approximately unbiased (AU) p-value greater than 0.9. All analysis was performed in R 2.9.0 using in-house scripts.

### Cell Line Verification

STR (short tandem repeat) was used for profiling to verify authenticity of the panel of cell lines. We simultaneously amplified 8 STR loci, in a multiplex PCR reaction (Promega PowerPlex 1.2 System) and used the ATCC database for comparison, where possible. In addition, gain and loss of expression of particular subtype markers, described by Hollestelle et *al*.(14), were confirmed by western blot as well as microarray analysis.

### RESULTS

*Generation of functional viability profiles of breast cancer* To generate functional profiles of breast cancer, we used an approach that involves high-throughput RNA interference/short interfering RNA (siRNA) viability screening of multiple cell lines and the integration of viability profiles with gene expression, genetic, genomic and histological analysis (3). We reasoned that siRNAs causing significant loss of cell viability in all of the cell lines assayed likely targeted genes that had an essential ubiquitous function in both normal and tumor cells. Similarly, siRNAs that had no significant effect on viability in any of the cell lines were either not functional or targeted non-essential genes. Finally, siRNAs that caused significant lethality in only some but not all cell lines likely identified genes that represented tumour-specific dependencies and candidate therapeutic targets.

To generate functional viability profiles for breast cancer we selected a panel of 34 breast cancer-derived cell lines and optimised these for high-throughput siRNA screening. Subsequently, each cell line was transfected with a 96 well-plate arrayed siRNA library targeting 714 kinases and kinase-related genes (see Methods). After five population doublings, cell viability in each well was estimated by use of a highly sensitive luminescent assay measuring cellular ATP levels. To identify loss of viability /inhibition/failure to proliferate effects in each cell line, luminescence readings from each well were log transformed and then centred by the plate median, to account for plate-to-plate variation. To allow data to be compared between different cell lines, plate-centred data from each screen was standardised by the use of a Z score statistic, where Z=0 represents no effect on viability and negative Z scores represent loss of viability. RNA interference screens were carried out in triplicate and comparison of Z score data from replica screens of each cell line showed the screening process to be highly robust.

Having screened and processed data from 34 breast cancer lines, we selected 20 for further analysis, based on stringent QC thresholds (such as Z prime-factor). This panel encapsulated each of the major breast cancer subtypes (4) and included hormone receptor (Estrogen Receptor (ER), Progesterone Receptor (PR)), ERBB2 positive as well as ER, PR and ERBB2 negative models (triple negative), as characterised by parallel immunohistochemical, fluorescent *in situ* hybridisation (FISH) and western blot analysis. To complement this functional viability profiling, we also characterised the breast tumor panel using transcript microarrays, array-based comparative genomic hybridisation (aCGH), drug sensitivity for a small number of targeted agents, as well as gene mutation data. In the siRNA screens we used a stringent threshold of Z<-2 for defining loss of viability effects (approximately equal to Q=0.02275 in one screen replica, and Q= 1.21 x10-5 in triplicate screens (5)) and identified 330 genes whose depletion by siRNA caused loss of viability in at least one cell line, and 180 genes that caused loss of viability in two or more cell lines. This analysis enabled us to identify the predominant dependencies in each cell line.

### Identification of de facto genetic dependencies using functional viability profiling

The delineation of functional viability profiles for the breast cell line panel provided a framework for identifying dependencies in particular genetically-defined subgroups of the disease. As proof of this principle, we used supervised hierarchical clustering of the viability data and integration with DNA sequence, aCGH, FISH and western blot data to identify additional gene dependencies in *PIK3CA* mutant or *ERBB2* amplified cell lines. Somatic mutations of *PIK3CA,* which encodes the p110α catalytic subunit of PI3-kinase, have been shown to induce oncogenic transformation *in vitro* and *in vivo (6). PIK3CA* mutations are found in 8% - 35% of human breast cancers making them one of the most common genetic aberrations in this disease (7). Supervised hierarchical clustering of functional viability data according to *PIK3CA* mutation status identified 30 siRNAs that caused loss of viability preferentially in the *PIK3CA* mutant subgroup (Fig. 1a). Of 714 genes profiled in the cell line panel, the highest ranked *PIK3CA* mutant selective effect was targeting of *PIK3CA* itself (p=0.014; Fig. 1b). In six of seven *PIK3CA* mutant lines, *PIK3CA* siRNA gives effects of Z<-2, suggesting that *PIK3CA* addiction is one of the most significant effects in *PIK3CA* mutant tumor cells. Statistically, the chance probability, Q, of a *PIK3CA* siRNA effect of <-2 in six independently-derived *PIK3CA* mutant lines is approximately Q=1.39x10⁻¹⁰ (where Z -2 in one cell line is approximately equal to, a Q = 0.02275 effect, or 1 in 44; in six cell lines this is a Q = (0.02275)⁶ chance effect), demonstrating a strong dependency on *PIK3CA* in breast cancers with *PIK3CA* mutations despite their diverse genetic backgrounds. We also noted that *PIK3CA* mutant breast cancer lines were preferentially sensitive to *AKT2* and *AKT3* siRNAs (Fig. 1c). Although the mechanisms by which mutant *PIK3CA* mediates cellular transformation are not completely understood, it is likely that part of this effect is mediated by signalling through AKT (8). The preferential sensitivity of *PIK3CA* mutant cells to AKT targeting supported the hypothesis that the viability profiles had the ability to illuminate true addiction pathways.

Our analysis of the functional viability profiles based on *PIK3CA* mutation status also suggested that cell lines with mutations in the kinase domain of *PIK3CA* tend to be more sensitive to *PIK3CA* siRNA targeting than cell lines harbouring non-kinase domain *PIK3CA* mutations. Whilst a wide variety of tumour specific *PIK3CA* mutations have been identified, the vast majority occur in two hotspots, either in the kinase domain (for example p.H1047R) or in the helical domain (e.g. p.E542K or p.E545K). It has been suggested that helical and kinase domain mutants have distinct physiologic phenotypes in human cells (9, 10), and the differential effects of *PIK3CA* targeting in helical vs. kinase domain mutants could also suggest differences in *PIK3CA* addiction.

Interrogation of the functional profiles also identified other well-established oncogene addiction effects. For example, approximately 15% of breast cancers exhibit amplification and overexpression of the *ERBB2* gene and the addiction of some tumor types to ERBB2 signalling is the basis for the clinical success of ERBB2-targeting agents such as trastuzumab and lapatinib. Supervised clustering of viability data based on *ERBB2* amplification status (based upon an integration with aCGH and FISH data identified a number of genes that when silenced were selectively lethal in *ERBB2* amplified breast cancer lines (Fig. Id). One of the most dominant effects was targeting of *ERBB2* itself (*p*=0.003), supporting the hypothesis that *ERBB2* amplification can lead to an oncogene addiction effect (Fig. 1d,e). This reaffirms that our approach may have merit in identifying genes functionally relevant to tumor cell viability in clinically defined subgroups. Moreover many *ERBB2* amplified cell lines are highly sensitive to *PIK3CA* siRNA (Figure 1d) supporting the clinical development of PI3K inhibitors in ERBB2-dependent breast cancer regardless of *PIK3CA* mutation status.

### PTEN deficient breast tumor cells are dependent upon the mitotic checkpoint kinase TTK

On the basis of these proof-of-concept examples, we assessed whether functional viability profiles could be used to identify novel addictions/candidate therapeutic targets that could be used in specific genetic or phenotypic backgrounds. To do this, we annotated the viability data set with gene mutation, transcript expression and phenotypic data and then used hierarchical clustering of viability data to identify candidate gene dependencies in different genetic backgrounds. For example, integrating functional viability profiles with genomic profiles generated by aCGH profiling enabled the identification of candidate genetic dependencies for breast tumor cells carrying amplification events commonly found in breast cancer (11, 12). Similar to the ERBB2 paradigm, we identified candidate genetic dependencies associated with chromosome (chr.) 11q13.2-q13.3 amplification; the chr. 8q23.3-q24.3 amplification encompassing the *MYC* oncogene; amplification of *FGFR1* on chr. 8p12; amplification of *AURKA* on chr. 20q13; amplification of *CDK4* on chr. 12q14.1; the chr. 17q21-q23 amplification encompassing the *PPM1D* oncogene; and amplification of *MDM2* on chr. 12q14.3-q15. A similar analysis allowed us to identify candidate dependencies in models with common tumor suppressor or oncogene mutation events such as those for *PTEN* mutation, *CDKN2A (p16)* mutation, *KRAS* mutation, *p53* mutation, *BRCA1* mutation or *RB* mutation. Finally, we compared the functional viability profiles to transcript microarray defined subtypes of breast cancer (e.g. ERBB2, basal/triple negative, luminal status (13, 14)) to identify candidate dependencies in each group.

To validate our approach we selected two genetic dependencies for more detailed analysis. By comparing candidate genetic dependencies between *PTEN* mutant models and those with wild type PTEN expression (see Table 1 below), we observed the apparent dependency of *PTEN* mutant breast cancer lines for a series of genes controlling the mitotic spindle assembly checkpoint. The most significant amongst these was dependency on the *TTK protein kinase* gene (15) (aka *MPS1*) (p=0.0012; Fig. 2a,b). To directly address the hypothesis that TTK inhibition was selective for *PTEN* mutant cancer cells, we used isogenic cancer lines in which both copies of the *PTEN* gene had been rendered dysfunctional by gene targeting (16). We showed that multiple siRNAs silencing TTK (Fig. 2c, d)) or chemical inhibition of TTK (15) (using two distinct TTK inhibitors) was selective for PTEN deficiency (Fig. 2e,f), confirming the observations made in the more genetically diverse breast cancer line panel. This indicated that TTK inhibition may be a novel therapeutic strategy for treating PTEN mutant tumours. Aneuploidy is frequently observed in both human breast carcinomas with low expression of PTEN and prostatic intraepithelial neoplasia from Pten mutant mice (17). This latter phenomenon is perhaps explained by the centromeric dysfunction in PTEN mutant tumor cells, most likely mediated by a loss of the interaction between PTEN and CENP-C, a key kinetochore component (18). TTK is required for normal function of the mitotic spindle checkpoint and it is established that TTK inhibition drives early exit from mitosis and chromosomal aneuploidy. In tumor cells with an aneuploid phenotype TTK inhibition further exacerbates aneupliody and is particularly lethal (19). By examining metaphases from PTEN deficient tumor cells exposed to a small molecule TTK inhibitor, we showed that the frequency of abnormal metaphases was substantially increased in PTEN deficient cells, which may explain the PTEN deficient-selective effect of inhibiting this mitotic checkpoint kinase.

### Functional viability profiling identifies candidate functional taxonomies

Gene expression and genomic profiles have been used to sub-classify breast cancers. We investigated whether the patterns of response to gene silencing by siRNA could also be used for this purpose to define a "functional taxonomy". Hierarchical cluster analysis of transcript profiles classifies breast tumours and tumor cell lines into luminal and basal-like molecular subtypes (14). Hierarchical clustering of genes that when silenced caused loss of viability (Z<-2) in two or more cell lines revealed two distinct groups, distinct from those formed by the clustering of the expression data (Fig. 3.

Group 1 was enriched for breast cancer lines with *PTEN* mutations (5/5 *PTEN* mutant lines in group 1, p=0.0016), whilst Group 2 was enriched for *PIK3CA* mutant lines (5/7 *PIK3CA* mutant lines, *p*=0.038). CAL51 and MDAMB453, which carry both *PTEN* and *PIK3CA* mutations were classified into Group 1. Interestingly, although the *ERBB2* amplified cell lines were distributed evenly between the two groups, the cell lines resistant to *ERBB2* silencing and also lapatinib treatment were all contained within Group 1 (JIMT1, MDAMB453 and VP229) and those
sensitive to *ERBB2* silencing/lapatinib (HCC202, BT474 and SKBR3) fell into Group 2 (Fig. 3b). In general, the distinction between Groups 1 and 2 implies that our panel of breast cancer cell lines functionally divide into two groups according to their dependency on well-established essential cancer networks, and is independent from the currently used clinical and transcriptomically-defined breast cancer subgroups.

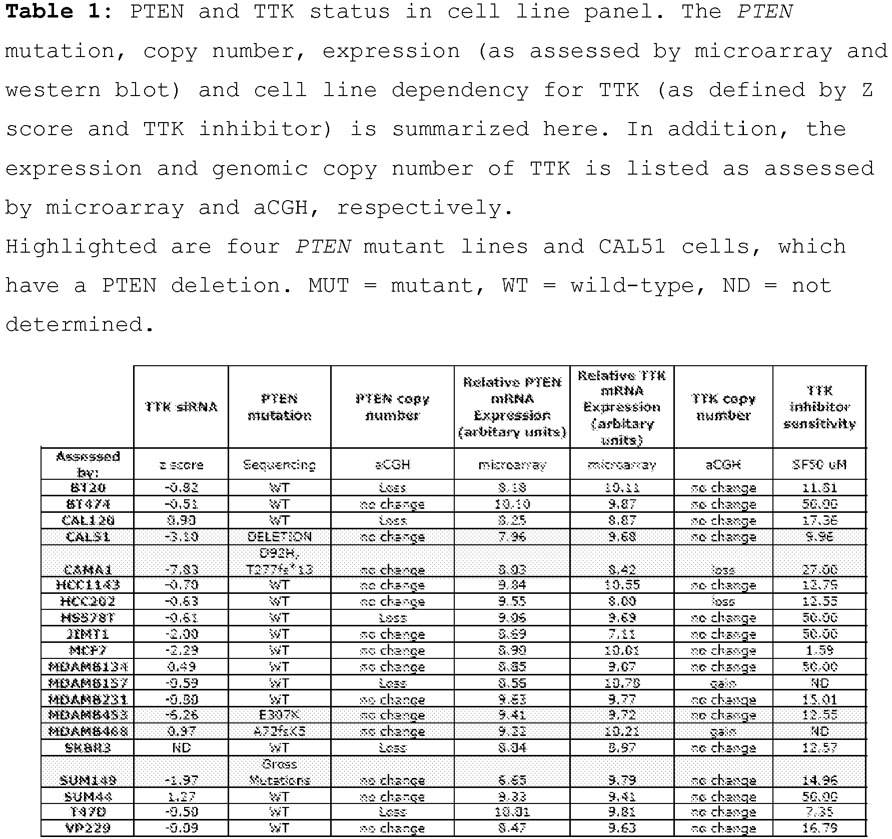

### References

1. Ashworth A, Lord CJ, Reis-Filho JS. Genetic interactions in cancer progression and treatment. Cell 2011;145:30-8.
2. Baselga J, Swain SM. Novel anticancer targets: revisiting ERBB2 and discovering ERBB3. Nat Rev Cancer 2009;9:463-75.
3. Iorns E, Lord CJ, Grigoriadis A, McDonald S, Fenwick K, Mackay A, et al. Integrated functional, gene expression and genomic analysis for the identification of cancer targets. PLoS One 2009;4:e5120.
4. Neve RM, Chin K, Fridlyand J, Yeh J, Baehner FL, Fevr T, et al. A collection of breast cancer cell lines for the study of functionally distinct cancer subtypes. Cancer Cell 2006;10:515-27.
5. Boutros M, Bras LP, Huber W. Analysis of cell-based RNAi screens. Genome Biol 2006;7:R66.
6. Zhao L, Vogt PK. Class I PI3K in oncogenic cellular transformation. Oncogene 2008;27:5486-96.
7. Stemke-Hale K, Gonzalez-Angulo AM, Lluch A, Neve RM, Kuo WL, Davies M, et al. An integrative genomic and proteomic analysis of PIK3CA, PTEN, and AKT mutations in breast cancer. Cancer Res 2008;68:6084-91.
8. Manning BD, Cantley LC. AKT/PKB signaling: navigating downstream. Cell 2007;129:1261-74.
9. Zhao L, Vogt PK. Helical domain and kinase domain mutations in p110alpha of phosphatidylinositol 3-kinase induce gain of function by different mechanisms. Proc Natl Acad Sci U S A 2008;105:2652-7.
10. Pang H, Flinn R, Patsialou A, Wyckoff J, Roussos ET, Wu H, et al. Differential enhancement of breast cancer cell motility and metastasis by helical and kinase domain mutations of class IA phosphoinositide 3-kinase. Cancer Res 2009;69:8868-76.
11. Natrajan R, Weigelt B, Mackay A, Geyer FC, Grigoriadis A, Tan DS, et al. An integrative genomic and transcriptomic analysis reveals molecular pathways and networks regulated by copy number aberrations in basal-like, HER2 and luminal cancers. Breast Cancer Res Treat 2010;121:575-89.
12. Santarius T, Shipley J, Brewer D, Stratton MR, Cooper CS. A census of amplified and overexpressed human cancer genes. Nat Rev Cancer 2010;10:59-64.
13. Sorlie T, Perou CM, Tibshirani R, Aas T, Geisler S, Johnsen H, et al. Gene expression patterns of breast carcinomas distinguish tumor subclasses with clinical implications. Proc Natl Acad Sci U S A 2001;98:10869-74.
14. Hollestelle A, Nagel JH, Smid M, Lam S, Elstrodt F, Wasielewski M, et al. Distinct gene mutation profiles among luminal-type and basal-type breast cancer cell lines. Breast Cancer Res Treat;121:53-64.
15. Hewitt L, Tighe A, Santaguida S, White AM, Jones CD, Musacchio A, et al. Sustained Mps1 activity is required in mitosis to recruit O-Mad2 to the Mad1-C-Mad2 core complex. J Cell Biol 2010;190:25-34.
16. Lee C, Kim JS, Waldman T. PTEN gene targeting reveals a radiation-induced size checkpoint in human cancer cells. Cancer Res 2004;64:6906-14.
17. Puc J, Parsons R. PTEN loss inhibits CHK1 to cause double stranded-DNA breaks in cells. Cell Cycle 2005;4:927-9.
18. Shen WH, Balajee AS, Wang J, Wu H, Eng C, Pandolfi PP, et al. Essential role for nuclear PTEN in maintaining chromosomal integrity. Cell 2007;128:157-70.
19. Kwiatkowski N, Jelluma N, Filippakopoulos P, Soundararajan M, Manak MS, Kwon M, et al. Small-molecule kinase inhibitors provide insight into Mps1 cell cycle function. Nat Chem Biol 2010;6:359-68.
20. Lagier-Tourenne C, Tazir M, Lopez LC, Quinzii CM, Assoum M, Drouot N, et al. ADCK3, an ancestral kinase, is mutated in a form of recessive ataxia associated with coenzyme Q10 deficiency. Am J Hum Genet 2008;82:661-72.
21. Sotiriou C, Pusztai L. Gene-expression signatures in breast cancer. N Engl J Med 2009;360:790-800.
22. Wang Y, Klijn JG, Zhang Y, Sieuwerts AM, Look MP, Yang F, et al. Geneexpression profiles to predict distant metastasis of lymph-node-negative primary breast cancer. Lancet 2005;365:671-9.
23. Sims D, Bursteinas B, Gao Q, Jain E, Mackay A, Mitsopoulos C, et al. ROCK: a breast cancer functional genomics resource. Breast Cancer Res Treat 2010.
24. Zhang JH, Chung TD, Oldenburg KR. A Simple Statistical Parameter for Use in Evaluation and Validation of High Throughput Screening Assays. J Biomol Screen 1999;4:67-73.
25. Iorns E, Turner NC, Elliott R, Syed N, Garrone O, Gasco M, et al. Identification of CDK10 as an important determinant of resistance to endocrine therapy for breast cancer. Cancer Cell 2008;13:91-104.
26. Turner NC, Lord CJ, Iorns E, Brough R, Swift S, Elliott R, et al. A synthetic lethal siRNA screen identifying genes mediating sensitivity to a PARP inhibitor. EMBO J 2008;27:1368-77.
27. Farmer H, McCabe N, Lord CJ, Tutt AN, Johnson DA, Richardson TB, et al. Targeting the DNA repair defect in BRCA mutant cells as a therapeutic strategy. Nature 2005;434:917-21.
28. Natrajan R, Weigelt B, Mackay A, Geyer FC, Grigoriadis A, Tan DS, et al. An integrative genomic and transcriptomic analysis reveals molecular pathways and networks regulated by copy number aberrations in basal-like, HER2 and luminal cancers. Breast Cancer Res Treat;121:575-89.
29. Geyer FC, Weigelt B, Natrajan R, Lambros MB, de Biase D, Vatcheva R, et al. Molecular analysis reveals a genetic basis for the phenotypic diversity of metaplastic breast carcinomas. J Pathol;220:562-73.
30. Hochberg Y, Benjamini Y. More powerful procedures for multiple significance testing. Stat Med 1990;9:811-8.
31. Natrajan R, Lambros MB, Rodriguez-Pinilla SM, Moreno-Bueno G, Tan DS, Marchio C, et al. Tiling path genomic profiling of grade 3 invasive ductal breast cancers. Clin Cancer Res 2009;15:2711-22.

## Claims

1. An inhibitor of TTK protein kinase for use in a method of treating an individual having cancer by inhibiting said TTK protein kinase, wherein the cancer is a Phosphatase and Tensin Homolog (PTEN) mutated or deficient cancer.

2. The inhibitor of TTK protein kinase for use in a method of treatment according to claim 1 wherein the PTEN deficient cancer is PTEN null or the PTEN mutated cancer comprises a truncating mutation or one or more substitutions.

3. The inhibitor of TTK protein kinase for use in a method of treatment according to any one of the preceding claims, wherein the PTEN mutant or deficient cancer affects the autonomic ganglia, biliary tract, bone, breast, CNS, cervix, endometrium, eye, haematopoietic and lymphoid tissue, kidney, large intestine, liver, lung, meninges, oesophagus, ovary, pancreas, prostate, salivary gland, skin, soft tissue, stomach, testis, thyroid, upper aerodigestive tract, urinary tract, or vulva.

4. The inhibitor of TTK protein kinase for use in a method of treatment according to any one of claims 1 to 3, wherein the PTEN mutant or deficient cancer is breast cancer, endometrial carcinoma, glioblastoma, prostate cancer, colon cancer or lung cancer.

5. The inhibitor of TTK protein kinase for use in a method of treatment according to any one of the preceding claims, wherein:
(a) the inhibitor is a nucleic acid inhibitor, an antibody, a small molecule, such as AZ3146 or CCT132774, or a peptide; or
(b) the inhibitor is a nucleic acid inhibitor which is a RNAi molecule or a siRNA molecule or an shRNA molecule.

6. The inhibitor of TTK protein kinase for use in a method of treatment according to any one of the preceding claims, wherein treatment with an mitotic kinase inhibitor is combined with a further anti-cancer therapy, optionally wherein treatment with mitotic kinase inhibitor is used in conjunction with a further chemotherapeutic agent.

7. The inhibitor of TTK protein kinase for use in a method of treatment according to claim 6, wherein the further chemotherapeutic agent is Amsacrine (Amsidine), Bleomycin, Busulfan, Capecitabine (Xeloda), Carboplatin, Carmustine (BCNU), Chlorambucil(Leukeran), Cisplatin, Cladribine(Leustat), Clofarabine (Evoltra), Crisantaspase (Erwinase), Cyclophosphamide, Cytarabine (ARA-C), Dacarbazine (DTIC), Dactinomycin (Actinomycin D),Daunorubicin, Docetaxel (Taxotere), Doxorubicin, Epirubicin, Etoposide (Vepesid, VP-16), Fludarabine (Fludara), Fluorouracil (5-FU), Gemcitabine (Gemzar), Hydroxyurea (Hydroxycarbamide, Hydrea), Idarubicin (Zavedos). Ifosfamide (Mitoxana), Irinotecan (CPT-11, Campto), Leucovorin (folinic acid), Liposomal doxorubicin (Caelyx, Myocet), Liposomal daunorubicin (DaunoXome®) Lomustine, Melphalan, Mercaptopurine, Mesna, Methotrexate, Mitomycin, Mitoxantrone, Oxaliplatin (Eloxatin), Paclitaxel (Taxol), Pemetrexed (Alimta), Pentostatin (Nipent), Procarbazine, Raltitrexed (Tomudex®), Streptozocin (Zanosar®), Tegafur-uracil (Uftoral), Temozolomide (Temodal), Teniposide (Vumon), Thiotepa, Tioguanine (6-TG) (Lanvis), Topotecan (Hycamtin), Treosulfan, Vinblastine (Velbe), Vincristine (Oncovin), Vindesine (Eldisine) or Vinorelbine (Navelbine).

8. The inhibitor of TTK protein kinase for use in a method of treatment according to claim 6 or claim 7, wherein the further chemotherapeutic agent is 5FU, a BCL-XL inhibitor, a BCR-ABL, cKIT or PDGFR inhibitor, a CDK inhibitor, a CHK inhibitor, a COX2 inhibitor, a EGFR inhibitor, a HER2 targeted agent, a HSP inhibitor, a hTERT inhibitors, an IDO inhibitor, a MDM2 targeted agent, Methotrexate, a mTOR inhibitor, a PARP inhibitor, a PI3K inhibitor, a platinum salt, a proteasome inhibitor, a RAR or RXR inhibitor, a SRC inhibitor, a TGFB2 inhibitor, a topoisomerase inhibitor, a VEGF, RAF, cKIT or PDGFR inhibitor, or a SMC mimetic.

9. The inhibitor of TTK protein kinase for use in a method of treatment according to any one of the preceding claims, wherein the method comprises the step of determining whether the individual has a PTEN mutated or deficient cancer.

10. The inhibitor of TTK protein kinase for use in a method of treatment according to any one of the preceding claims, wherein determining whether the individual has PTEN mutated or deficient cancer comprises measuring PTEN protein expression in a sample obtained from the individual to determine whether the PTEN protein is mutated or deficient;
and optionally wherein determining PTEN protein expression comprises one or more of determining PTEN protein expression in a tumour sample using immunohistochemistry, determining PTEN protein expression comprises measuring PTEN protein levels in a cell lysate by ELISA or Western blotting, and/or determining PTEN protein expression comprises using a binding agent capable of specifically binding to the PTEN protein, or a fragment thereof.

11. The inhibitor of TTK protein kinase for use in a method of treatment according to any one of the preceding claims, wherein determining whether the individual has a PTEN mutated or deficient cancer is performed on genomic nucleic acid extracted from a sample of cells obtained from the breast cancer or from a sample of cancer cells circulating in blood;
and optionally wherein determining the expression of the PTEN gene comprises extracting RNA from a sample of cancer cells and measuring expression by real time PCR and/or by using a probe capable of hybridising to PTEN RNA, the probe optionally being immobilised in a microarray.

12. The inhibitor of TTK protein kinase for use in a method of treatment according to any one of the preceding claims, wherein the method comprises the initial step of obtaining a sample from said individual, optionally wherein the sample is a tumour sample, a blood sample, a tissue sample or a cell sample.

13. A method of screening for agents useful in the treatment of a PTEN mutated or deficient cancer, the method employing first and second cell lines, wherein the first cell line is PTEN and the second cell line is PTEN proficient, the method comprising:
(a) contacting the first and second mammalian cell lines with at least one candidate agent;
(b) determining the amount of cell death in the first and second cell lines;
(c) selecting a candidate agent which is synthetically lethal in the first cell line; and
(d) determining whether the candidate agent selected in step (c) is an inhibitor of TTK protein kinase.

14. A method of screening for agents useful in the treatment of PTEN mutated or deficient cancer, the method comprising:
(a) contacting TTK protein kinase with at least one candidate agent;
(b) determining an effect of the at least one candidate agent on an activity of the TTK protein kinase; and
(c) selecting a candidate agent that inhibits the activity of the mitotic kinase as being useful for the treatment of the PTEN mutated or deficient cancer.

15. The method according to claim 14, further comprising the step of contacting a candidate agent selected in step (c) with a PTEN mutated or deficient cancer cell line to determine whether the candidate agent is cytotoxic to the cancer cell line.

16. The method according to claim 14 or claim 15, wherein:
(a) the candidate agent is a candidate nucleic acid inhibitor, a candidate antibody or a candidate small molecule or a candidate peptide; and/or
(b) the candidate agent is a compound that is part of a compound library, and optionally wherein the candidate compound has a molecular weight of less than 100 Da; and/or
(c) the candidate agent or candidate compounds is a drug approved for use in the treatment of cancer.

17. The method according to any one of claims 13 to 16, further comprising determining whether a candidate agent is not lethal on normal cells and/or determining the effect of combinations of two or more candidate compounds on the cell lines or protein targets.

## Patentansprüche

1. TTK-Proteinkinase-Inhibitor zur Verwendung in einem Behandlungsverfahren eines Individuums mit Krebs durch die Inhibierung der TTK-Proteinkinase, wobei der Krebs ein PTEN- (Phosphatase und Tensin-Homolog) mutierter oder -defizienter Krebs ist.

2. TTK-Proteinkinase-Inhibitor zur Verwendung in einem Behandlungsverfahren nach Anspruch 1, wobei der PTEN-defiziente Krebs PTEN-Null ist oder der PTEN-mutierte Krebs eine trunkierende Mutation oder eine oder mehrere Substitutionen umfasst.

3. TTK-Proteinkinase-Inhibitor zur Verwendung in einem Behandlungsverfahren nach einem der vorangegangenen Ansprüche, wobei der PTEN-mutierte oder -defiziente Krebs den/die/das autonomen Ganglien, Gallenwege, Knochen, Brust, ZNS, Gebärmutterhals, Endometrium, Auge, hämatopoetische und lymphoide Gewebe, Niere, Dickdarm, Leber, Lunge, Meninges, Ösophagus, Eierstock, Bauchspeicheldrüse, Prostata, Speicheldrüsen, Haut, Weichgewebe, Magen, Hoden, Schilddrüsen, oberen Aerodigestivtrakt, Harntrakt oder Vulva betrifft.

4. TTK-Proteinkinase-Inhibitor zur Verwendung in einem Behandlungsverfahren nach einem der Ansprüche 1 bis 3, wobei der PTEN-mutierte oder -defiziente Krebs Brustkrebs, Endometriumkarzinom, Glioblastom, Prostatakrebs, Darmkrebs oder Lungenkrebs ist.

5. TTK-Proteinkinase-Inhibitor zur Verwendung in einem Behandlungsverfahren nach einem der vorangegangenen Ansprüche, wobei:
(a) der Inhibitor ein Nucleinsäureinhibitor, ein Antikörper, ein kleines Molekül wie z.B. AZ3146 oder CCT132774 oder ein Peptid ist; oder
(b) der Inhibitor ein Nucleinsäureinhibitor ist, der ein RNAi-Molekül oder ein siRNA-Molekül oder ein shRNA-Molekül ist.

6. TTK-Proteinkinase-Inhibitor zur Verwendung in einem Behandlungsverfahren nach einem der vorangegangenen Ansprüche, wobei die Behandlung mit einem mitotischen Kinaseinhibitor mit einer weiteren Antikrebstherapie kombiniert wird, wobei die Behandlung mit dem mitotischen Kinaseinhibitor gegebenenfalls in Verbindung mit einem weiteren Chemotherapeutikum verwendet wird

7. TTK-Proteinkinase-Inhibitor zur Verwendung in einem Behandlungsverfahren nach Anspruch 6, wobei das weitere Chemotherapeutikum Amsacrin (Amsidin), Bleomycin, Busulfan, Capecitabin (Xeloda), Carboplatin, Carmustin (BCNU), Chlorambucil (Leukeran), Cisplatin, Cladribin (Leustat), Clofarabin (Evoltra), Crisantaspase (Erwinase), Cyclophosphamid, Cytarabin (ARA-C), Dacarbazin (DTIC), Dactinomycin (Actinomycin D), Daunorubicin, Docetaxel (Taxotere), Doxorubicin, Epirubicin, Etoposid (Vepesid, VP-16), Fludarabin (Fludara), Fluoruracil (5-FU), Gemcitabin (Gemzar), Hydroxyharnstoff (Hydroxycarbamid, Hydrea), Idarubicin (Zavedos), Ifosfamid (Mitoxana), Irinotecan (CPT-11, Campto), Leucovorin (Folinsäure), Liposomales Doxorubicin (Caelyx, Myocet), Liposomales Daunorubicin (DaunoXome®), Lomustin, Melphalan, Mercaptopurin, Mesna, Methotrexat, Mitomycin, Mitoxantron, Oxaliplatin (Eloxatin), Paclitaxel (Taxol), Pemetrexed (Alimta), Pentostatin (Nipent), Procarbazin, Raltitrexed (Tomudex®), Streptozocin (Zanosar®), Tegafur-Uracil (Uftoral), Temozolomid (Temodal), Teniposid (Vumon), Thiotepa, Tioguanin (6-TG) (Lanvis), Topotecan (Hycamtin), Treosulfan, Vinblastin (Velbe), Vincristin (Oncovin), Vindesin (Eldisin) oder Vinorelbin (Navelbin) ist.

8. TTK-Proteinkinase-Inhibitor zur Verwendung in einem Behandlungsverfahren nach Anspruch 6 oder Anspruch 7, wobei das weitere Chemotherapeutikum 5FU, ein BCL-XL-Inhibitor, ein BCR-ABL-, cKIT- oder PDGFR-Inhibitor, ein CDK-Inhibitor, ein CHK-Inhibitor, ein COX2-Inhibitor, ein EGFR-Inhibitor, ein gegen HER2 gerichtetes Mittel, ein HSP-Inhibitor, ein hTERT-Inhibitor, an IDO-Inhibitor, ein gegen MDM2 gerichtetes Mittel, Methotrexat, ein mTOR-Inhibitor, ein PARP-Inhibitor, ein PI3K-Inhibitor, ein Platinsalz, ein Proteasom-Inhibitor, ein RAR- oder RXR-Inhibitor, ein SRC-Inhibitor, ein TGFB2-Inhibitor, ein Topoisomerase-Inhibitor, ein VEGF-, RAF-, cKIT- oder PDGFR-Inhibitor oder ein SMC-Mimetikum ist.

9. TTK-Proteinkinase-Inhibitor zur Verwendung in einem Behandlungsverfahren nach einem der vorangegangenen Ansprüche, wobei das Verfahren den Schritt des Bestimmens, ob das Individuum einen PTEN-mutierten oder -defizienten Krebs aufweist, umfasst.

10. TTK-Proteinkinase-Inhibitor zur Verwendung in einem Behandlungsverfahren nach einem der vorangegangenen Ansprüche, wobei das Bestimmen, ob das Individuum PTEN-mutierten oder -defizienten Krebs aufweist, das Messen der PTEN-Proteinexpression in einer von dem Individuum erhaltenen Probe umfasst, um zu bestimmen, ob das PTEN-Protein mutiert oder defizient ist;
und wobei gegebenenfalls das Bestimmen der PTEN-Proteinexpression eines oder mehrere aus dem Bestimmen der PTEN-Proteinexpression in einer Tumorprobe unter Verwendung von Immunhistochemie, Bestimmen der PTEN-Proteinexpression umfassend das Messen der PTEN-Proteinspiegel in einem Zelllysat mittels ELISA oder Western-Blot und/oder Bestimmen der PTEN-Proteinexpression umfassend die Verwendung eines Bindemittels, das in der Lage ist, spezifisch an das PTEN-Protein oder ein Fragment davon zu binden, umfasst.

11. TTK-Proteinkinase-Inhibitor zur Verwendung in einem Behandlungsverfahren nach einem der vorangegangenen Ansprüche, wobei das Bestimmen, ob das Individuum PTEN-mutierten oder -defizienten Krebs aufweist, auf einer genomischen Nucleinsäure durchgeführt wird, die aus einer Probe von Zellen, die aus dem Brustkrebs erhalten wurden, oder aus einer Probe von Krebszellen, die im Blut zirkulieren, extrahiert wurde;
und wobei gegebenenfalls das Bestimmen der Expression des PTEN-Gens das Extrahieren von RNA aus einer Probe von Krebszellen und das Messen der Expression mittels Echtzeit-PCR und/oder unter Verwendung einer Sonde, die in der Lage ist, an PTEN-RNA zu hybridisieren, umfasst, wobei die Sonde gegebenenfalls in einem Mikroarray immobilisiert wird.

12. TTK-Proteinkinase-Inhibitor zur Verwendung in einem Behandlungsverfahren nach einem der vorangegangenen Ansprüche, wobei das Verfahren den Ausgangsschritt des Erhaltens einer Probe von dem Individuum umfasst, wobei die Probe gegebenenfalls eine Tumorprobe, eine Blutprobe, eine Gewebeprobe oder eine Zellprobe ist.

13. Verfahren zum Screening auf Mittel, die in der Behandlung eines PTEN-mutierten oder -defizienten Krebses nützlich sind, wobei das Verfahren erste und zweite Zelllinien verwendet, wobei die erste Zelllinie PTEN ist und die zweite Zelllinie PTENkompetent ist, wobei das Verfahren Folgendes umfasst:
(a) Kontaktieren der ersten und zweiten Säugetierzelllinie mit zumindest einem Kandidatenmittel;
(b) Bestimmen der Menge an Zelltod in der ersten und zweiten Zelllinie;
(c) Auswählen eines Kandidatenmittels, das in der ersten Zelllinie synthetisch tödlich ist; und
(d) Bestimmen, ob das in Schritt (c) ausgewählte Kandidatenmittel ein TTK-Proteinkinase-Inhibitor ist.

14. Verfahren zum Screening auf Mittel, die in der Behandlung eines PTEN-mutierten oder -defizienten Krebses nützlich sind, wobei das Verfahren Folgendes umfasst:
(a) Kontaktieren einer TTK-Proteinkinase mit zumindest einem Kandidatenmittel;
(b) Bestimmen einer Wirkung des zumindest einen Kandidatenmittels auf eine Aktivität der TTK-Proteinkinase; und
(c) Auswählen eines Kandidatenmittels, das die Aktivität der mitotischen Kinase hemmt, als nützlich für die Behandlung von PTEN-mutiertem oder -defizientem Krebs.

15. Verfahren nach Anspruch 14, das weiters den Schritt des Kontaktierens eines Kandidatenmittels, das in Schritt (c) ausgewählt wurde, mit einer PTEN-mutierten oder -defizienten Krebszelllinie umfasst, um zu bestimmen, ob das Kandidatenmittel für die Krebszelllinie zytotoxisch ist.

16. Verfahren nach Anspruch 14 oder Anspruch 15, wobei:
(a) das Kandidatenmittel ein Kandidaten-Nucleinsäure-Inhibitor, ein Kandidaten-Antikörper oder ein kleines Kandidatenmolekül oder ein Kandidatenpeptid ist; und/oder
(b) das Kandidatenmittel eine Verbindung ist, die Teil einer Verbindungsbibliothek ist, wobei die Kandidatenverbindung gegebenenfalls ein Molekulargewicht von weniger als 100 Da aufweist; und/oder
(c) das/die Kandidatenmittel oder Kandidatenverbindungen ein Arzneimittel ist/sind, das/die für die Verwendung in der Behandlung von Krebs zugelassen ist/sind.

17. Verfahren nach einem der Ansprüche 13 bis 16, das weiters das Bestimmen, ob ein Kandidatenmittel für normale Zellen nicht tödlich ist, und/oder das Bestimmen der Wirkung von Kombinationen aus zwei oder mehreren Kandidatenverbindungen auf die Zelllinien oder Protein-Targets umfasst.

## Revendications

1. Inhibiteur de protéine kinase TTK pour utilisation dans un procédé de traitement d'un individu ayant un cancer par inhibition de ladite protéine kinase TTK, dans lequel le cancer est un cancer à mutation ou à déficience de PTEN (phosphatase et homologue de la tensine).

2. Inhibiteur de protéine kinase TTK pour utilisation dans un procédé de traitement selon la revendication 1, dans lequel le cancer à déficience de PTEN est sans PTEN ou le cancer à mutation de PTEN comprend une mutation de troncature ou une ou plusieurs substitutions.

3. Inhibiteur de protéine kinase TTK pour utilisation dans un procédé de traitement selon l'une quelconque des revendications précédentes, dans lequel le cancer à mutation ou à déficience de PTEN affecte les ganglions autonomes, le tractus biliaire, les os, les seins, le SNC, le col de l'utérus, l'endomètre, les yeux, les tissus hématopoïétiques et lymphoïdes, les reins, le gros intestin, le foie, les poumons, les méninges, l'oesophage, les ovaires, le pancréas, la prostate, les glandes salivaires, la peau, les tissus mous, l'estomac, les testicules, la thyroïde, les voies aérodigestives supérieures, le tractus urinaire ou la vulve.

4. Inhibiteur de protéine kinase TTK pour utilisation dans un procédé de traitement selon l'une quelconque des revendications 1 à 3, dans lequel le cancer à mutation ou à déficience de PTEN est un cancer du sein, un carcinome de l'endomètre, un glioblastome, un cancer de la prostate, un cancer du côlon ou un cancer du poumon.

5. Inhibiteur de protéine kinase TTK pour utilisation dans un procédé de traitement selon l'une quelconque des revendications précédentes, dans lequel :
(a) l'inhibiteur est un inhibiteur d'acide nucléique, un anticorps, une petite molécule, telle que AZ3146 ou CCT132774, ou un peptide ; ou
(b) l'inhibiteur est un inhibiteur d'acide nucléique qui est une molécule d'ARNi ou une molécule de petit ARN interférent ou une molécule d'ARNsh.

6. Inhibiteur de protéine kinase TTK pour utilisation dans un procédé de traitement selon l'une quelconque des revendications précédentes, dans lequel le traitement par un inhibiteur de kinase mitotique est combiné avec une autre thérapie anticancéreuse, facultativement dans lequel le traitement par inhibiteur de kinase mitotique est utilisé conjointement avec un autre agent chimiothérapeutique.

7. Inhibiteur de protéine kinase TTK pour utilisation dans un procédé de traitement selon la revendication 6, dans lequel l'autre agent chimiothérapeutique est l'amsacrine (Amsidine), la bléomycine, le busulfan, la capécitabine (Xeloda), le carboplatine, la carmustine (BCNU), le chlorambucil (Leukeran), le cisplatine, la cladribine (Leustat), la clofarabine (Evoltra), le crisantaspase (Erwinase), le cyclophosphamide, la cytarabine (ARA-C), la dacarbazine (DTIC), la dactinomycine (Actinomycine D), la daunorubicine, le docétaxel (Taxotère), la doxorubicine, l'épirubicine, l'étoposide (Vépéside, VP-16), la fludarabine (Fludara), le fluorouracile (5-FU), la gemcitabine (Gemzar), l'hydroxyurée (Hydroxycarbamide, Hydréa), l'idarubicine (Zavedos), l'ifosfamide (Mitoxana), l'irinotécan (CPT-11, Campto), la leucovorine (acide folinique), la doxorubicine liposomale (Caelyx, Myocet), la daunorubicine liposomale (DaunoXome®), la lomustine, le melphalan, la mercaptopurine, le mesna, le méthotrexate, la mitomycine, la mitoxantrone, l'oxaliplatine (Éloxatine), le paclitaxel (Taxol), le pémétrexed (Alimta), la pentostatine (Nipent), la procarbazine, le raltitrexed (Tomudex®), la streptozocine (Zanosar®), le tégafur-uracile (Uftoral), le témozolomide (Temodal), le téniposide (Vumon), le thiotépa, la tioguanine (6-TG) (Lanvis), le topotécan (Hycamtin), le tréosulfan, la vinblastine (Velbe), la vincristine (Oncovin), la vindésine (Eldisine) ou la vinorelbine (Navelbine).

8. Inhibiteur de protéine kinase TTK pour utilisation dans un procédé de traitement selon la revendication 6 ou la revendication 7, dans lequel l'autre agent chimiothérapeutique est le 5FU, un inhibiteur de BCL-XL, un inhibiteur de BCR-ABL, de cKIT ou de PDGFR, un inhibiteur de CDK, un inhibiteur de CHK, un inhibiteur de COX2, un inhibiteur d'EGFR, un agent ciblé par HER2, un inhibiteur d'HSP, un inhibiteur d'hTERT, un inhibiteur d'IDO, un agent ciblé par MDM2, le méthotrexate, un inhibiteur de mTOR, un inhibiteur de PARP, un inhibiteur de PI3K, un sel de platine, un inhibiteur de protéasome, un inhibiteur de RAR ou de RXR, un inhibiteur de SRC, un inhibiteur de TGFB2, un inhibiteur de topoisomérase, un inhibiteur de VEGF, de RAF, de cKIT ou de PDGFR, ou un mimétique de SMC.

9. Inhibiteur de protéine kinase TTK pour utilisation dans un procédé de traitement selon l'une quelconque des revendications précédentes, dans lequel le procédé comprend l'étape consistant à déterminer si l'individu a un cancer à mutation ou à déficience de PTEN.

10. Inhibiteur de protéine kinase TTK pour utilisation dans un procédé de traitement selon l'une quelconque des revendications précédentes, dans lequel l'opération consistant à déterminer si l'individu a un cancer à mutation ou à déficience de PTEN comprend la mesure de l'expression de la protéine PTEN dans un échantillon obtenu auprès de l'individu pour déterminer si la protéine PTEN est mutée ou déficiente ;
et facultativement dans lequel la détermination de l'expression de la protéine PTEN comprend une ou plusieurs de la détermination de l'expression de la protéine PTEN dans un échantillon de tumeur à l'aide d'une immunohistochimie, la détermination de l'expression de la protéine PTEN comprend la mesure des taux de protéine PTEN dans un lysat cellulaire par ELISA ou buvardage de western, et/ou la détermination de l'expression de la protéine PTEN comprend l'utilisation d'un agent de liaison capable de se lier spécifiquement à la protéine PTEN, ou à un fragment de celle-ci.

11. Inhibiteur de protéine kinase TTK pour utilisation dans un procédé de traitement selon l'une quelconque des revendications précédentes, dans lequel l'opération consistant à déterminer si l'individu a un cancer à mutation ou à déficience de PTEN est effectuée sur un acide nucléique génomique extrait à partir d'un échantillon de cellules obtenu à partir du cancer du sein ou à partir d'un échantillon de cellules cancéreuses circulant dans le sang ;
et facultativement dans lequel la détermination de l'expression du gène PTEN comprend l'extraction d'ARN à partir d'un échantillon de cellules cancéreuses et la mesure de l'expression par PCR en temps réel et/ou par utilisation d'une sonde capable de s'hybrider avec l'ARN de PTEN, la sonde étant facultativement immobilisée dans un microréseau.

12. Inhibiteur de protéine kinase TTK pour utilisation dans un procédé de traitement selon l'une quelconque des revendications précédentes, dans lequel le procédé comprend l'étape initiale d'obtention d'un échantillon auprès dudit individu, facultativement dans lequel l'échantillon est un échantillon de tumeur, un échantillon de sang, un échantillon de tissu ou un échantillon de cellules.

13. Procédé de criblage d'agents utiles dans le traitement d'un cancer à mutation ou à déficience de PTEN, le procédé employant une première et une seconde lignée cellulaire, dans lequel la première lignée cellulaire est PTEN et la seconde lignée cellulaire est capable de produire PTEN, le procédé comprenant :
(a) la mise en contact de la première et de la seconde lignée cellulaire de mammifère avec au moins un agent candidat ;
(b) la détermination de la quantité de mort cellulaire dans la première et la seconde lignée cellulaire ;
(c) la sélection d'un agent candidat qui est synthétiquement létal dans la première lignée cellulaire ; et
(d) l'opération consistant à déterminer si l'agent candidat sélectionné à l'étape (c) est un inhibiteur de protéine kinase TTK.

14. Procédé de criblage d'agents utiles dans le traitement d'un cancer à mutation ou à déficience de PTEN, le procédé comprenant :
(a) la mise en contact d'une protéine kinase TTK avec au moins un agent candidat ;
(b) la détermination d'un effet de l'au moins un agent candidat sur une activité de la protéine kinase TTK ; et
(c) la sélection d'un agent candidat qui inhibe l'activité de la kinase mitotique comme étant utile pour le traitement du cancer à mutation ou à déficience de PTEN.

15. Procédé selon la revendication 14, comprenant en outre l'étape de mise en contact d'un agent candidat sélectionné à l'étape (c) avec une lignée cellulaire de cancer à mutation ou à déficience de PTEN pour déterminer si l'agent candidat est cytotoxique pour la lignée cellulaire cancéreuse.

16. Procédé selon la revendication 14 ou la revendication 15, dans lequel :
(a) l'agent candidat est un inhibiteur d'acide nucléique candidat, un anticorps candidat ou une petite molécule candidate ou un peptide candidat ; et/ou
(b) l'agent candidat est un composé qui fait partie d'une banque de composés, et facultativement dans lequel le composé candidat a un poids moléculaire inférieur à 100 Da ; et/ou
(c) l'agent candidat ou le composé candidat est un médicament dont l'utilisation est approuvée dans le traitement du cancer.

17. Procédé selon l'une quelconque des revendications 13 à 16, comprenant en outre l'opération consistant à déterminer si un agent candidat n'est pas létal sur les cellules normales et/ou à déterminer l'effet de combinaisons de deux composés candidats ou plus sur les lignées cellulaires ou les protéines cibles.
